# EUROPEAN PATENT APPLICATION

(11) **EP 4 792 824 A1**
(43) Date of publication of application: **19.08.2026**
(21) Application number: 25225358.8
(22) Date of filing: 19.12.2025
(51) Int. Cl.: A61K 38/16, A61K 47/64, A61L 27/22, A61L 27/54, A61P 21/00, A61P 21/04, C07K 14/00, C07K 19/00

(54) **PEPTIDE HYDROGELS AND THEIR USE FOR SKELETAL MUSCLE REGENERATION AS STIMULATORS OF MYOBLAST MIGRATION AND FUSION**

(30) Priority: 18.02.2025 EP 25461510; 26.04.2025 EP 25172768
(71) Applicant: Uniwersytet Warszawski, 00-927 Warszawa (PL); Uniwersytet Gdanski, 80-309 Gdansk (PL)
(72) Inventor: Ciemerych-Litwinienko, Maria, 02-132 Warszawa (PL); Archacka, Karolina, 05-816 Michalowice (PL); Bielak, Kacper, 05-092 Lomianki (PL); Brzoska-Wojtowicz, Edyta, 03-937 Warszawa (PL); Grabowska-Kowalik, Iwona, 02-384 Warszawa (PL); Janczyk-Ilach, Katarzyna, 03-757 Warszawa (PL); Kosmala, Magdalena, 02-927 Warszawa (PL); Kulig, Izabela, 84-300 Lebork (PL); Michalska, Zuzanna, 03-226 Warszawa (PL); Morawska, Agnieszka, 03-680 Warszawa (PL); Morytz, Justyna, 03-252 Warszawa (PL); Ostaszewska, Anna, 02-791 Warszawa (PL); Streminska, Wladyslawa, 02-372 Warszawa (PL); Trusiak, Hanna, 15-345 Bia ystok (PL); Zimowska-Wypych, Malgorzata, 01-627 Warszawa (PL); Rodziewicz-Motowidlo, Sylwia, 83-010 Rotmanka (PL); Fularczyk, Martyna, 80-293 Gdansk (PL); Dzierzynska, Maria, 81-824 Sopot (PL); Kuropka, Anna, 02-107 Warszawa (PL); Lukaszewicz, Julia, 01-234 Warszawa (PL); Piorkowska, Gabriela, 26-803 Promna (PL); Pluta, Alicja, 05-500 Piaseczno (PL); Ploska, Wanda, 00-225 Warszawa (PL); Romanska, Aleksandra, 02-760 Warszawa (PL); Skorupinski, Piotr, 05-270 Marki (PL); Charzynska, Marta, 02-679 Warszawa (PL); Panasiuk, Martyna, 00-215 Warszawa (PL)
(74) Representative: Czarnik, Maciej

(57) **Abstract**

A peptide-based hydrogel for skeletal muscle regeneration, comprising: a) a self-assembling RADA16-I peptide forming a nanofibrous hydrogel matrix; and b) at least one bioactive peptide selected from: peptide fragment of stromal cell-derived factor 1 (SDF-1): SEQ ID NO: 1, SEQ ID NO: 2, SEQ ID NO: 5, SEQ ID NO: 6, or peptide fragment of interleukin-4 (IL-4): SEQ ID NO: 3, SEQ ID NO: 4, SEQ ID NO: 7, SEQ ID NO: 8, wherein the bioactive peptide is covalently linked to the RADA16-I sequence via a matrix metalloproteinase (MMPs) cleavable peptide linker and wherein cleavage of the linker by an MMPs results in controlled, localized release of the bioactive peptide in the microenvironment of regenerating muscle tissue.

## Description

The present invention relates to the field of regenerative medicine, tissue engineering, and biomaterials for skeletal muscle repair. More particularly, the invention concerns peptide-based hydrogels functionalized with bioactive fragments of stromal cell-derived factor 1 (SDF-1) or interleukin-4 (IL-4), which are covalently attached to a self-assembling RADA16-I peptide scaffold through matrix metalloproteinase (MMP)-cleavable linkers. The invention further relates to compositions comprising such hydrogels, including compositions containing human pluripotent stem cell-derived myoblasts, and to medical and therapeutic uses of these hydrogels for the regeneration of injured or degenerating skeletal muscle tissue.

### Background of the Invention

Skeletal muscle regeneration is governed by the presence of satellite cells (SCs) - a unique population of quiescent, unipotent muscle stem cells that activate and proliferate upon muscle injury. Activated SCs generate committed myoblasts, which differentiate into myocytes, fuse into multinucleated myotubes, and ultimately mature into new muscle fibers (myofibers). In addition to SCs, several other cell types (including endothelial cells, neural cells, and immune cells) participate in muscle repair. These supportive cells are essential for clearing debris and promoting revascularization and re-innervation during the later stages of regeneration [1].

Under certain conditions, however, the intrinsic regenerative capacity of skeletal muscle is compromised. For example, in cases of severe trauma or volumetric muscle loss (VML), or in degenerative muscle diseases such as muscular dystrophy, muscle reconstruction may be incomplete or inefficient. Such impaired regeneration is often accompanied by chronic inflammation and fibrotic scar formation in the muscle tissue [2]. Moreover, factors like advanced age or the pathological environment of diseased muscle can reduce the number and functionality of satellite cells, further limiting the muscle's natural ability to regenerate [3]. To overcome these obstacles, various therapeutic strategies are being explored. Among them, approaches based on biomaterial scaffolds have shown particular promise. Hydrogels offer numerous beneficial features for tissue engineering applications - they are typically biocompatible, and their physical and biochemical properties can be readily tailored to support cell growth and tissue repair [4-5].

Many types of hydrogels have been tested as potential treatments for muscle injuries or degenerative conditions, demonstrating positive effects on muscle regeneration [6]. For example, Borselli *et al.* reported that injection of an alginate hydrogel loaded with growth factors (VEGF and IGF-1) into injured muscle led to increased muscle weight and enhanced muscle fiber reconstruction (e.g. larger fiber diameter and improved force generation), as well as greater muscle vascularization and innervation [7]. This illustrates the potential of hydrogel-based delivery of bioactive molecules to improve muscle healing. However, many prior studies have not fully elucidated the mechanisms by which hydrogels exert their pro-regenerative effects. In particular, there is often a lack of detailed analysis regarding how hydrogel influences the various cell populations in regenerating muscle. Understanding these cell-specific interactions is important for rational design of more effective biomaterials.

Another challenge in muscle repair is preventing aberrant outcomes of dysregulated regeneration. If muscle healing is improperly regulated (for instance, after severe injury, burns, or repetitive trauma), pathological tissue changes can occur. Examples include heterotopic ossification (HO), where bone tissue forms in muscles or surrounding soft tissue, as well as excessive fibrosis or calcification of muscle, or unwanted adipose tissue accumulation in the muscle. Indeed, satellite cells and myoblasts themselves have been identified as potential progenitors for HO under such conditions. [15]. Fibrosis and ectopic calcification not only impede proper muscle function but can also significantly reduce the success of regenerative therapies. These considerations underscore the need for therapeutic approaches that promote muscle regeneration while also modulating the muscle environment to avoid deleterious outcomes like scarring or inappropriate tissue lineage differentiation.

In light of the above, improved biomaterial solutions are being pursued. The present inventors have focused on self-assembling peptide hydrogels based on the RADA16-I sequence, functionalized with specific peptide fragments that mimic critical muscle regulatory factors. In particular, two protein factors are of interest: interleukin-4 (IL-4) and stromal cell-derived factor-1 (SDF-1). IL-4 plays a crucial role in muscle regeneration by promoting the fusion of myogenic cells (it recruits mononucleated myoblasts/myocytes to fuse into growing myotubes) [8]. It has also been reported to enhance myoblast migration by upregulating integrin expression, and to favourably modulate myoblast metabolism (e.g. improving insulin signalling, GLUT4 translocation, and glucose uptake) [9-10]. SDF-1, on the other hand, is a chemokine produced by bone marrow and many tissues (including skeletal muscle) that is key for cell migration and homing. In the context of muscle, SDF-1 is known to attract circulating stem/progenitor cells to sites of injury [11]. The inventors' previous studies have shown that SDF-1 can induce up-regulation of CD9, a cell surface protein important for myogenic cell adhesion and fusion. These findings suggest that providing SDF-1 signals in injured muscle could enhance the recruitment and fusion competence of muscle cells.

While whole proteins like IL-4 or SDF-1 could in theory be delivered via biomaterials, the use of large proteins carries risks such as immune or inflammatory reactions. Therefore, the hydrogels in the present invention are modified with precisely selected peptide fragments of IL-4 and SDF-1, rather than the full proteins. Using peptide fragments significantly decreases the risk of an adverse immune response, as compared to hydrogels functionalized with entire proteins [12]. Each peptide fragment contains the bioactive portion of the cytokine of interest, capable of eliciting the desired cellular response (myoblast fusion/migration for IL-4, or cell homing for SDF-1), yet is small enough to minimize immunogenicity. Importantly, the custom-designed IL-4 and SDF-1 peptides are tethered to the RADA16-I hydrogel through short amino acid linker sequences that are substrates for specific matrix metalloproteinases (MMPs). MMPs are enzymes present in the muscle injury environment (secreted by muscle cells, fibroblasts, inflammatory cells, etc.) that can cleave extracellular matrix components. By incorporating MMP-cleavable linkers, the hydrogel is endowed with a controlled release mechanism: the attached IL-4 or SDF-1 peptide will remain bound and inactive until the appropriate MMPs are present to cleave the linker, thereby triggering release of the active peptide *in situ.* This strategy ensures that the therapeutic peptides are delivered in a localized and injury-responsive manner - predominantly at sites and times where regenerating muscle tissue produces MMPs - rather than being released systemically or all at once.

The aim of the invention is to provide novel peptide-based hydrogels, in which specially synthesized peptides exert a positive effect on muscle-regenerative cell types involved in muscle repair, in particular by enhancing cell survival (reducing apoptosis), proliferation, migratory capacity, and differentiation status, as well as by inducing beneficial changes in gene-expression profiles under these culture conditions. Another aim of the invention is to demonstrate their positive impact on induced pluripotent stem cell-derived myoblasts and their potential therapeutic application in muscle-regeneration treatments.

In summary, the background of this invention combines insights from muscle biology and biomaterials engineering. There is a clear need for interventions that can enhance muscle regeneration by targeting key cellular processes (such as myoblast migration and fusion) while avoiding complications like inflammation or fibrosis. Peptide-based hydrogels functionalized with MMP-sensitive IL-4 and SDF-1 fragments represent a novel solution addressing this need. Such hydrogels act as a scaffold to support cell growth and tissue formation, while simultaneously serving as a delivery vehicle for regenerative signals that are only activated in the presence of the muscle's own remodelling enzymes. This invention builds on prior knowledge of IL-4 and SDF-1's roles in myogenesis, and leverages the advantages of peptides and enzymatically responsive materials to create a sophisticated therapeutic platform for skeletal muscle repair.

Although the field of hydrogel biomaterials has rapidly expanded, there are comparatively few examples of peptide-based hydrogels specifically designed to promote muscle regeneration. This scarcity underscores an unmet need for bioactive, biomimetic scaffold capable of guiding muscle repair through controlled cellular and molecular mechanisms. Peptide hydrogels, with their modular design and self-assembling properties, have emerged as promising candidates to address these challenges. Their tunable composition allows for precise adjustment of mechanical strength, degradation rate, and biochemical functionality, making them adaptable to the dynamic environment of regenerating muscle tissue. Moreover, these hydrogels can mimic key features of the extracellular matrix (ECM) providing both structural and signalling cues that facilitate muscle cell adhesion, proliferation, differentiation, and integration with host tissue. Recent advances explored peptide-functionalized hydrogels capable of supporting muscle cell adhesion, proliferation, and differentiation.

In the state of the art, there are many patent documents related to hydrogel scaffolds.

For example, US2016030629 AA discloses peptide-albumin hydrogels having a self-assembling, 3-dimensional nanofiber matrix. The nanofiber matrix comprises an amphiphilic peptide and albumin. The peptide comprises a terminal hydrophobic region, a central turning region, and a terminal hydrophilic region. The patent application also discloses methods of making such hydrogels are also described, along with methods of using the hydrogels as scaffolding for tissue engineering, as 3-dimensional cell cultures, and for drug delivery, encapsulation of active agents (therapeutic cells, molecules, drugs, compounds), cell transplantation, cell storage, virus culture and storage.

In US2018023049 AA is disclosed a synthetic peptide hydrogel solution having a pH level of about 3.5 or less and a tonicity within an isotonic osmolality range for use in cell culture experimentation. The combination of these properties allows this hydrogel to better mimic natural conditions for cells, leading to more accurate and reliable experimental results.

A novel composition comprising self-assembling peptide hydrogels and cardiovascular system cells has been disclosed in US2004242469 AA. According to certain embodiments of this invention the endothelial cells form capillary-like structures. The endothelial cell-gel matrix serves as a pre-vascularized scaffold that may be used to culture additional cell types. Formation of mature blood vessels in or on the scaffold involves addition of smooth muscle cells and/or fibroblasts. Compositions of the invention may be used for cell culture and for administration to a subject to treat a variety of conditions including myocardial dysfunction or damage.

### Summary of the Invention

The present invention provides novel peptide-based hydrogels that release bioactive IL-4 and SDF-1 mimicking peptides in a controlled fashion in response to MMPs activity, and their use in stimulating muscle regeneration. The hydrogels are based on the self-assembling peptide RADA16-I (also referred to as "R" hydrogel), which forms a nanofibrous scaffold. Two series of hybrid hydrogels have been engineered by linking the RADA16-I sequence with custom peptides as follows:
- R1-series hydrogels: RADA16-I linked via a cleavable MMP-7 substrate motif to the active peptide fragment. Specifically, a tetrapeptide sequence Val-Leu-Gly-Leu (VLGL) was used as the MMP-7-sensitive linker. The IL-4 or SDF-1 fragment (denoted "-IL-4-X", "-IL-4-Y", "-SDF-1-X", or "-SDF-1-Y") is attached after this linker. For example, SEQ ID NO: 1 (R1-SDF-1-X) represents a hydrogel where the SDF-1-derived peptide "X" is connected to RADA16-I via the VLGL linker (which can be cleaved by MMP-7 to release the SDF-1 peptide).
- R4-series hydrogels: RADA16-I linked via a glycine spacer (GGG) and a MMP-2 substrate motif to the active peptide. In this design, the linker is a sequence recognized by MMP-2 (and related MMPs); in the exemplified hydrogels a Pro-Ala-Gly-Leu (PAGL) sequence is used. Thus, SEQ ID NO: 8 (R4-IL-4-Y) indicates a hydrogel where the IL-4 peptide "Y" is connected to RADA16-I through a triglycine + PAGL linker, allowing the IL-4 fragment to be cleaved and released by MMP-2 or similar enzymes present in the environment.

In both series, the notation "X" and "Y" after IL-4 or SDF-1 refers to two different custom-designed peptide fragments derived from the respective protein (previously described in the literature [14]). The designed peptide sequences are listed in Table 1 of the application. All peptides were synthesized and formulated into hydrogels separately, then mixed in a 1:1 ratio with RADA16-I hydrogel to form the final hybrid hydrogels. This mixing strategy allows the RADA16-I nanofibers and the functional peptide fibers to co-assemble, yielding a homogeneous hybrid network. Importantly, control hydrogels were also prepared for comparison: RADA16-I coupled with an RGD peptide (Arg-Gly-Asp) was made for both series (denoted R1-RGD and R4-RGD). RGD is a well-known cell-adhesion motif present in extracellular matrix proteins like fibronectin [13]. Including RGD-functionalized hydrogels as controls permits distinguishing the general effect of cell-adhesion peptide addition from the specific effects of IL-4 or SDF-1 peptides.

**Table 1**

| Code | Sequence | Mass Av. [Da] |
|---|---|---|
| R1-SDF-1-X (SEQ ID NO: 1) | RADARADARADARADAVLGLKPVSLSYR | 3024.63 |
| R1-SDF-1-Y (SEQ ID NO: 2) | RADARADARADARADAVLGLVARLKNNNRQV | 3386.84 |
| R1-IL-4-X (SEQ ID NO: 3) | RADARADARADARADAVLGLITLQEIIKTLN | 3360.85 |
| R1-IL-4-Y (SEQ ID NO: 4) | RADARADARADARADAVLGLIRFLKRLDRNLWG | 4003.22 |
| R4-SDF-1-X (SEQ ID NO: 5) | RADARADARADARADAGGGPAGLKPVSLSYR | 3151.63 |
| R4-SDF-1-Y (SEQ ID NO: 6) | RADARADARADARADAGGGPAGLVARLKNNNRQV | 3513.84 |
| R4-IL-4-X (SEQ ID NO: 7) | RADARADARADARADAGGGPAGLITLQEIIKTLN | 3487.86 |
| R4-IL-4-Y (SEQ ID NO: 8) | RADARADARADARADAGGGPAGLIRFLKRLDRNLWG | 4130.22 |
| RADA16-I / R | RADARADARADARADA | 1712.80 |
| R1 | RADARADARADARADAVLGL | 2095.31 |
| R4 | RADARADARADARADAGGGPAGL | 2222.37 |
| R1-RGD | RADARADARADARADAVLGLRGD | 2423.63 |
| R4-RGD | RADARADARADARADAGGGPAGLRGD | 2550.69 |

| | | |
|---|---|---|
| N-terminal acetylated, C-terminal amidated | | |

Overall, the invention encompasses eight exemplary peptide-functionalized hydrogels: SEQ ID NO: 3 (R1-IL-4-X), SEQ ID NO: 4 (R1-IL-4-Y), SEQ ID NO: 1 (R1-SDF-1-X), SEQ ID NO: 2 (R1-SDF-1-Y), and their R4-series counterparts SEQ ID NO: 7 (R4-IL-4-X), SEQ ID NO: 8 (R4-IL-4-Y), SEQ ID NO: 5 (R4-SDF-1-X), SEQ ID NO: 6 (R4-SDF-1-Y). These hydrogels each comprise the RADA16-I scaffold with a respective IL-4 or SDF-1 fragment peptide tethered via an MMP-cleavable linker. By deploying different linker sequences (MMP-7-sensitive in R1 vs MMP-2-sensitive in R4) and different peptide fragments (X or Y variants), the invention provides a versatile platform where the timing and localization of IL-4/SDF-1 release can be tailored to the enzymatic profile of the target tissue. The linker peptides (e.g., VLGL and PAGL) were chosen based on known MMPs cleavage specificity; thus, MMP-7 in the regenerating muscle will selectively cleave and release peptides from the R1 hydrogels, while MMP-2 (and also MMP-3, MMP-9, MMP-13, MMP-14) can cleave the linkers in R4 hydrogels. This design ensures that active IL-4 or SDF-1 mimetics are released only in the presence of the appropriate MMPs, effectively linking the peptide delivery to the natural healing process (since MMP-2 and MMP-7 are upregulated during muscle regeneration and remodelling).

The essence of the invention is a peptide-based hydrogel for skeletal muscle regeneration, comprising:
a) a self-assembling RADA16-I peptide forming a nanofibrous hydrogel matrix; and
b) at least one bioactive peptide selected from:
   peptide fragment of stromal cell-derived factor 1 (SDF-1): SEQ ID NO: 1, SEQ ID NO: 2, SEQ ID NO: 5, SEQ ID NO: 6, or
   peptide fragment of interleukin-4 (IL-4): SEQ ID NO: 3, SEQ ID NO: 4, SEQ ID NO: 7, SEQ ID NO: 8,
wherein the bioactive peptide is covalently linked to the RADA16-I sequence via a matrix metalloproteinase (MMPs) cleavable peptide linker and wherein cleavage of the linker by an MMPs results in controlled, localized release of the bioactive peptide in the microenvironment of regenerating muscle tissue.

Preferably, the bioactive peptide in the peptide hydrogel of the invention is SEQ ID NO: 1 or SEQ ID NO: 8.

Preferably, the matrix metalloproteinase cleavable peptide linker in the peptide hydrogel of the invention is selected from:
- the MMP-7-sensitive linker VLGL, or
- the MMP-2 or MMP-3 or MMP-9 or MMP-13 or MMP-14 sensitive linker GGG-PAGL.

Preferably, the linker in the peptide hydrogel of the invention is:
- VLGL and the hydrogel is an R1-type hydrogel; or
- GGG-PAGL and the hydrogel is an R4-type hydrogel.

The invention also relates to a composition comprising: (a) the hydrogel according to any of claims 1-6, and (b) myoblasts derived from human pluripotent stem cells, for implantation into damaged skeletal muscle tissue.

In another aspect, the invention relates to the peptide hydrogel of the invention or the composition comprising peptides of the invention for use as a medicament, in particular:
to use in the treatment of skeletal muscle injury or degeneration; or
to use in the treatment of a condition selected from: acute skeletal muscle injury, volumetric muscle loss (VML), muscular dystrophy, age-related sarcopenia, cachexia-associated muscle wasting, post-surgical or post-traumatic muscle regeneration, muscle atrophy due to immobilization or disuse.

The subject matter of the invention is presented in examples that do not limit its scope and in a drawing in which:

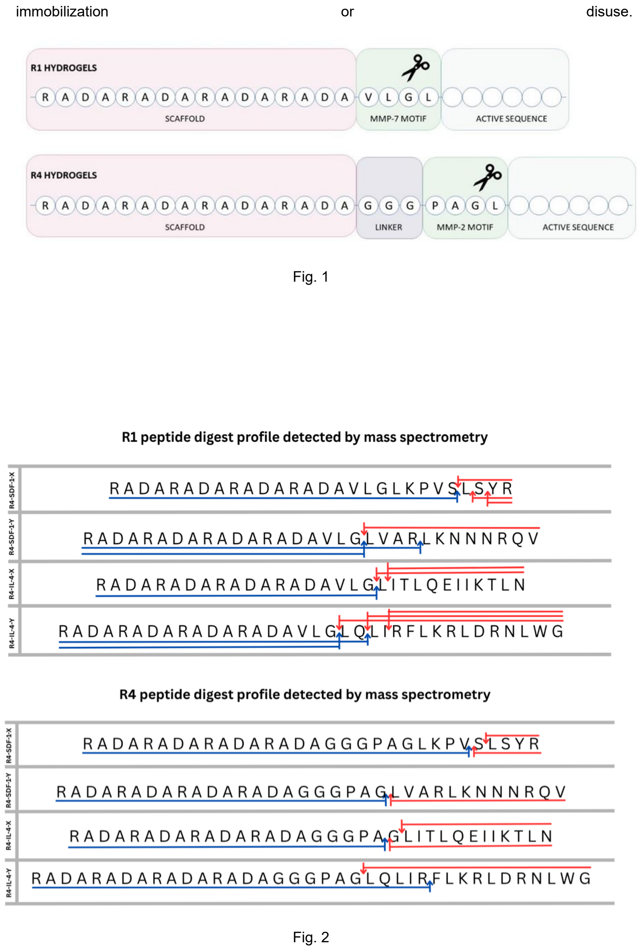

- Fig. 1.: illustrates hybrid hydrogel scheme.
- Fig. 2.: illustrates mass spectrometry analysis of R1 and R4 peptide digestion profiles after MMPs cleavage. Peptide fragments derived from the R1 and R4 hybrid hydrogels were analyzed by mass spectrometry following enzymatic digestion by matrix metalloproteinases. The identified peptide sequences are shown, with detected cleavage sites indicated by arrows. Blue arrows denote N-terminal cleavage sites, and red arrows indicate C-terminal cleavage sites. The underlined sequences represent the peptide fragments detected in the mass spectra.

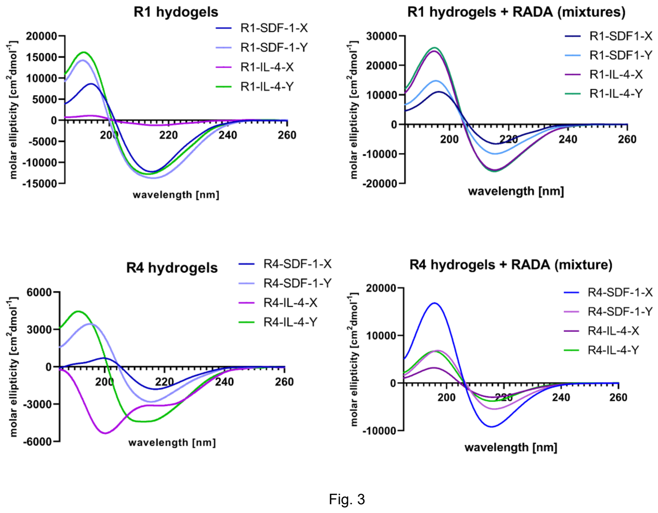

- Fig. 3.: illustrates circular dichroism spectra of R1 and R4 hybrid hydrogels and their mixtures with RADA16-I. CD spectra were recorded in the wavelength range 185-260 nm at room temperature for R1 and R4 hybrid hydrogels and their mixtures with RADA16-I at peptide concentration of 0.2 mg/ml, obtained by dilution after gelation (initial concentration 10 mg/ml). The spectra exhibit the characteristic negative band at 218 nm and positive band 196 nm, indicating a β-sheet-rich secondary structure of RADA16-I based self-assembling peptides. Minor spectral differences among samples reflect variations in peptide sequence and interactions influencing β-sheet content and supramolecular organization.

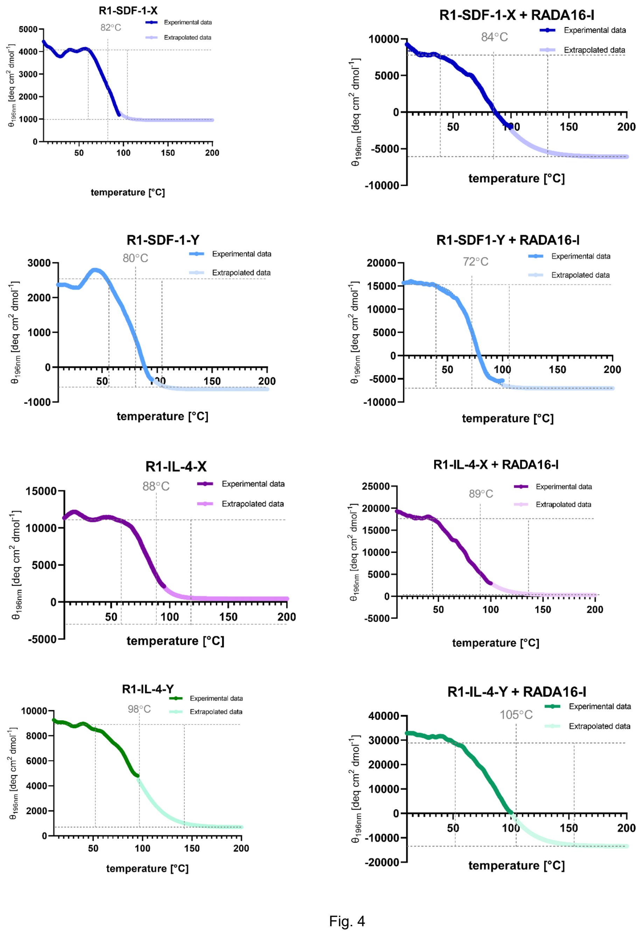

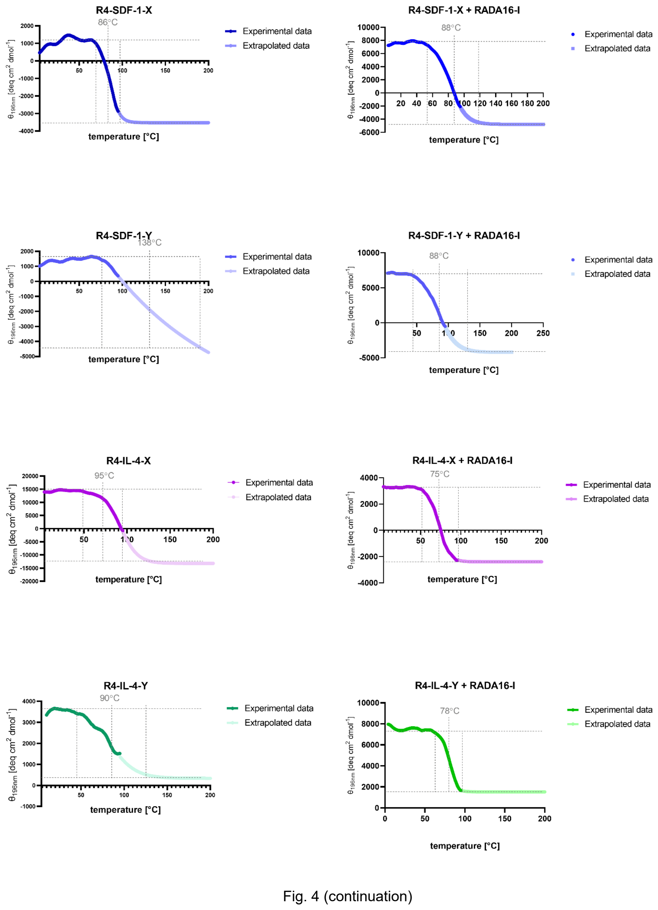

- Fig. 4.: illustrates thermal stability of the secondary structure of R1 and R4 hybrid hydrogels and their mixtures with RADA16-I measured by circular dichroism (CD). Temperature-dependent CD measurements at fixed wavelength of 196 nm were performed to assess the thermal stability of β-sheet structures in R1 and R4 hybrid hydrogels and their mixtures with RADA16-I. Experimental (dark lines) and extrapolated (lighter lines) data are shown. The midpoint transition temperatures (Tm) indicate the thermal unfolding of the β-sheet assemblies, with Tm values ranging from 72 to 105 °C depending on the peptide composition.

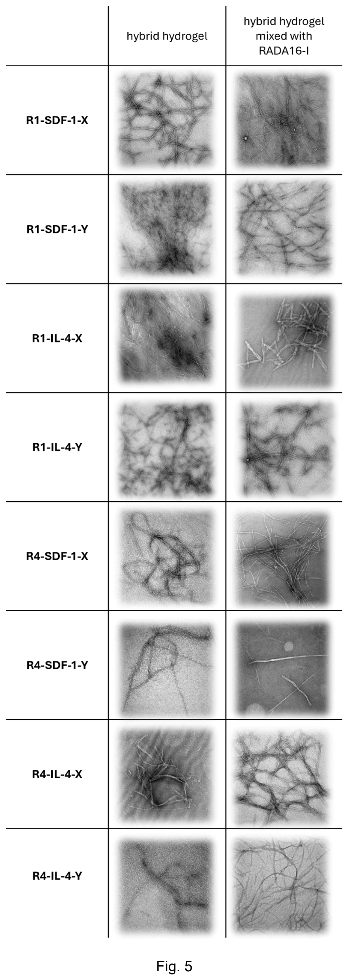

- Fig. 5.: comprises transmission electron microscopy (TEM) images of R1 and R4 hybrid hydrogels and their mixtures with RADA16-I. Representative TEM micrographs show the nanofibrous morphology of R1 and R4 hybrid peptide hydrogels, as well as their composites with RADA16-I. All samples exhibit interconnected fibrillar networks characteristic of β-sheet-driven self-assembly. The pure R1 and R4 hybrids form dense and entangled nanofiber structures, whereas mixtures with RADA16-I display more homogenous and well-organized fibrillar architecture, suggesting cooperative assembly between the hybrid peptide and RADA16-I. The observed difference in fiber thickness and network density reflect variations in peptide composition and secondary structure stability, consistent with CD and thermal analysis. Scale bars represent 100 nm.

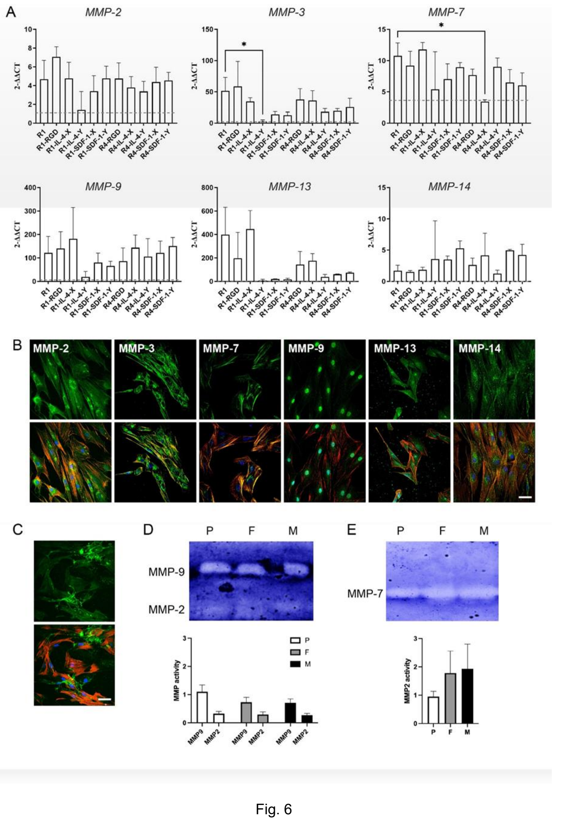

- Fig. 6.: Illustrates results of: A. qRT-PCR analysis of the expression of MMP-2, MMP-3, MMP-7, MMP-9, MMP-13, and MMP-14 in hSkMs at day 3 of culture on hydrogels: R, R1-RGD, SEQ ID NO: 3 (R1-IL-4-X), SEQ ID NO: 4 (R1-IL-4-Y), SEQ ID NO: 1 (R1-SDF-1-X), SEQ ID NO: 2 (R1-SDF-1-Y), R4-RGD, SEQ ID NO: 7 (R4-IL-4-X), SEQ ID NO: 8 (R4-IL-4-Y), SEQ ID NO: 5 (R4-SDF-1-X), SEQ ID NO: 6 (R4-SDF-1-Y). The gray line indicates the level of expression in cells maintained on plastic. Data are shown as mean ± SD. B. Immunolocalization of MMP-2, MMP-3, MMP-7, MMP-9, MMP-13, and MMP-14 proteins in hSkMs cultured on plastic. Nuclei are blue, actin is red, MMPs are green; scale bar = 50 µm. C. In situ zymography showing gelatinolytic activity of hSkMs on day 3 of culture. Nuclei are blue, actin is red, gelatinolytic activity is green. D. In-gel zymography detecting the activity of MMP-2 and MMP-7 in medium collected at proliferation (P), fusion (F), and myotube (M) stages of hSkM culture. E. In-gel zymography detecting the activity of MMP-2, MMP-7, and MMP-9 in medium collected at P, F, and M stages of hSkM culture. *p ≤ 0.05

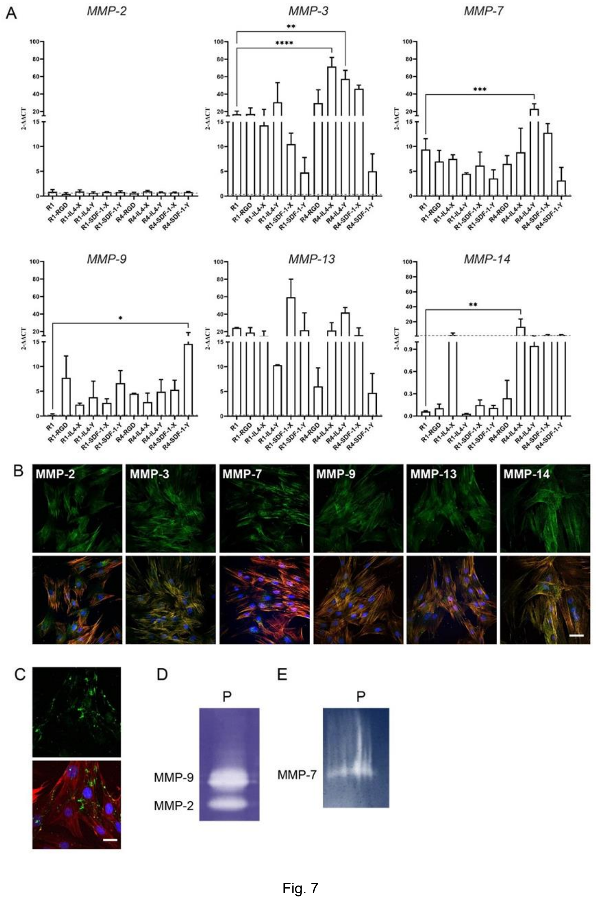

- Fig. 7.: Illustrates results of: A. qRT-PCR analysis of the expression of MMP-2, MMP-3, MMP-7, MMP-9, MMP-13, and MMP-14 in hDFs at day 3 of culture on hydrogels: R, R1-RGD, SEQ ID NO: 3 (R1-IL-4-X), SEQ ID NO: 4 (R1-IL-4-Y), SEQ ID NO: 1 (R1-SDF-1-X), SEQ ID NO: 2 (R1-SDF-1-Y), R4-RGD, SEQ ID NO: 7 (R4-IL-4-X), SEQ ID NO: 8 (R4-IL-4-Y), SEQ ID NO: 5 (R4-SDF-1-X), SEQ ID NO: 6 (R4-SDF-1-Y). The gray line indicates the level of expression in cells maintained on plastic. Data are mean ± SD. B. Immunolocalization of MMP-2, MMP-3, MMP-7, MMP-9, MMP-13, MMP-14 in hDFs cultured on plastic. Nuclei = blue, actin = red, MMPs = green; scale bar = 50 µm. C. In situ zymography showing gelatinolytic activity of hDFs on day 3 of culture. Nuclei are blue, actin is red, activity is green. D. In-gel zymography detecting the activity of MMP-2, MMP-7, and MMP-9 in medium collected after 3 days of hDF culture (proliferation stage, P). *p ≤ 0.05, ** p ≤ 0.01, *** p ≤ 0.001, *** p ≤ 0.0001.

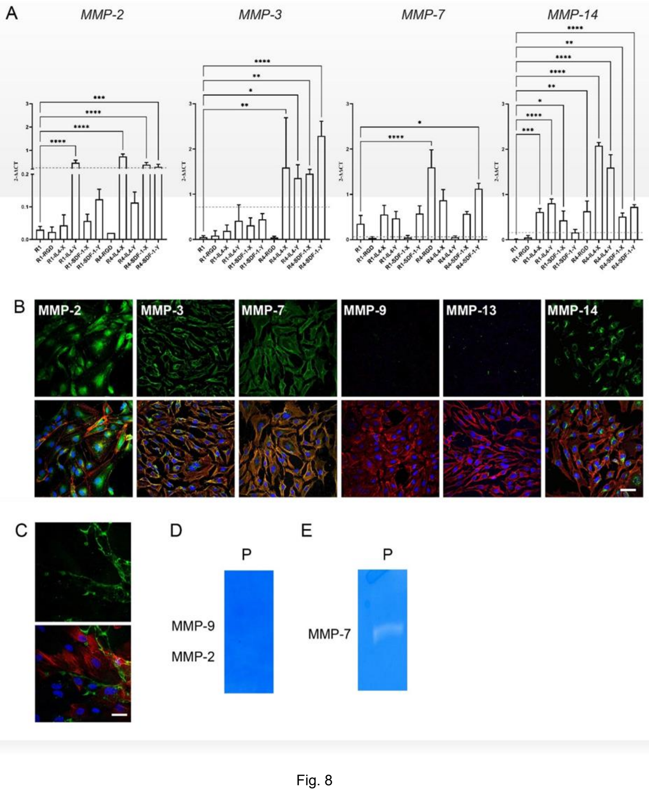

- Fig. 8.: Illustrates results of: A. qRT-PCR analysis of the expression of MMP-2, MMP-3, MMP-7, MMP-9, MMP-13, and MMP-14 in HUVECs at day 3 of culture on hydrogels: R, R1-RGD, SEQ ID NO: 3 (R1-IL-4-X), SEQ ID NO: 4 (R1-IL-4-Y), SEQ ID NO: 1 (R1-SDF-1-X), SEQ ID NO: 2 (R1-SDF-1-Y), R4-RGD, SEQ ID NO: 7 (R4-IL-4-X), SEQ ID NO: 8 (R4-IL-4-Y), SEQ ID NO: 5 (R4-SDF-1-X), SEQ ID NO: 6 (R4-SDF-1-Y). The gray line indicates the level of expression in cells on plastic. Data are mean ± SD. B. Immunolocalization of MMP-2, MMP-3, MMP-7, MMP-9, MMP-13, MMP-14 proteins in HUVECs cultured on plastic. Nuclei = blue, actin = red, MMPs = green; scale bar = 100 µm. C. In situ zymography showing gelatinolytic activity of HUVECs at day 3. Nuclei = blue, actin = red, activity = green. D-E. In-gel zymography analyzing the activity of MMP-2, MMP-7, and MMP-9 in medium collected after 3 days of HUVEC culture (proliferation stage, P). *p ≤ 0.05, ** p ≤ 0.01, *** p ≤ 0.001, **** p ≤ 0.0001.

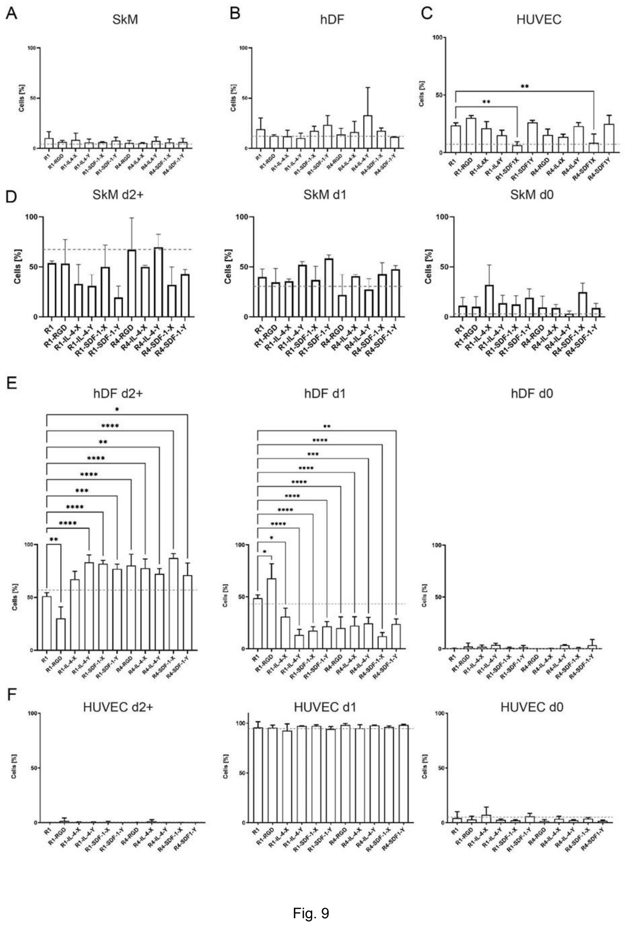

- Fig. 9.: illustrates results of proliferation and apoptosis assays using CFSE. A. Percentage of apoptotic cells in hSkMs cultures on hydrogel-coated surfaces over 72 h. Gray line indicates the level on plastic. B. Percentage of apoptotic cells in hDFs cultures on hydrogels over 72 h. Gray line indicates plastic. C. Percentage of apoptotic cells in HUVECs cultures on hydrogels over 72 h (p < 0.01 for SEQ ID NO: 1 (R1-SDF-1-X) vs R). D. Percentage of hSkMs undergoing two or more divisions (d2+), one division (d1), or none (d0) during 48 h culture on hydrogels. Gray line indicates the level on plastic (mean ± SD). E. Percentage of hDFs with d2+, d1, or d0 divisions during 48 h on hydrogels. Gray line indicates plastic (mean ± SD). F. Percentage of HUVECs with d2+, d1, or d0 divisions during 48 h on hydrogels. Gray line indicates plastic (mean ± SD). *p ≤ 0.05, ** p ≤ 0.01, *** p ≤ 0.001, **** p ≤ 0.0001.

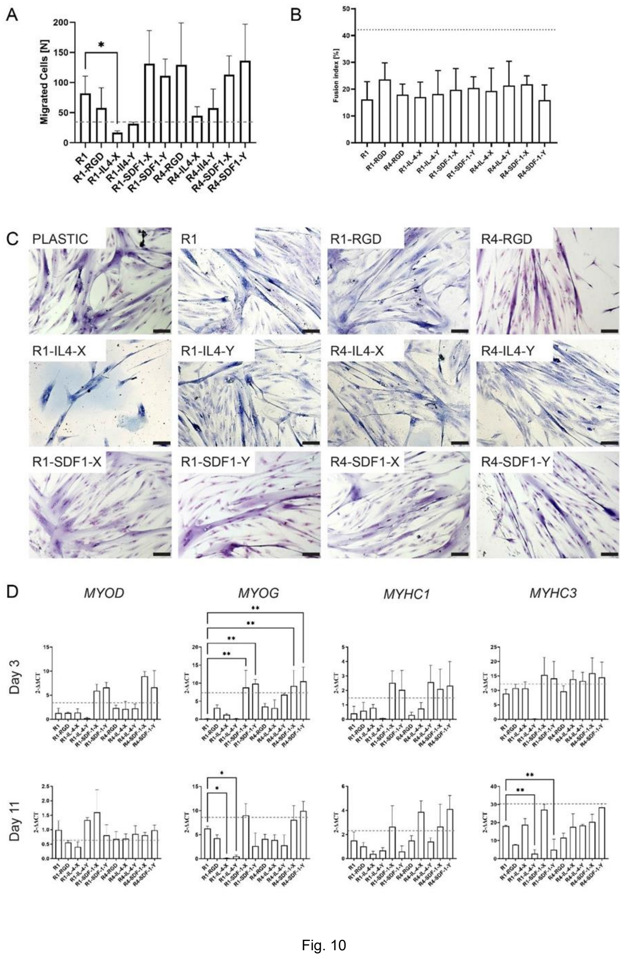

- Fig. 10.: illustrates results of: A. Migration assay of hSkMs: number of cells migrated through hydrogel-coated Transwells in 24 h. Gray line indicates the number migrated through an uncoated insert. B. Fusion index of hSkMs cultured on hydrogels (after 6 days differentiation). Gray line indicates the fusion index on plastic. C. Representative images of myotubes formed by hSkMs on hydrogels vs plastic (Giemsa-May Grünwald staining). D. qRT-PCR analysis of MYOD, MYOG, MYH1, MYH3 mRNA in hSkMs on hydrogels on day 3 (myoblast stage) and day 11 (post-differentiation). Gray line indicates expression in cells on plastic; *p ≤ 0.05, **p < 0.01.

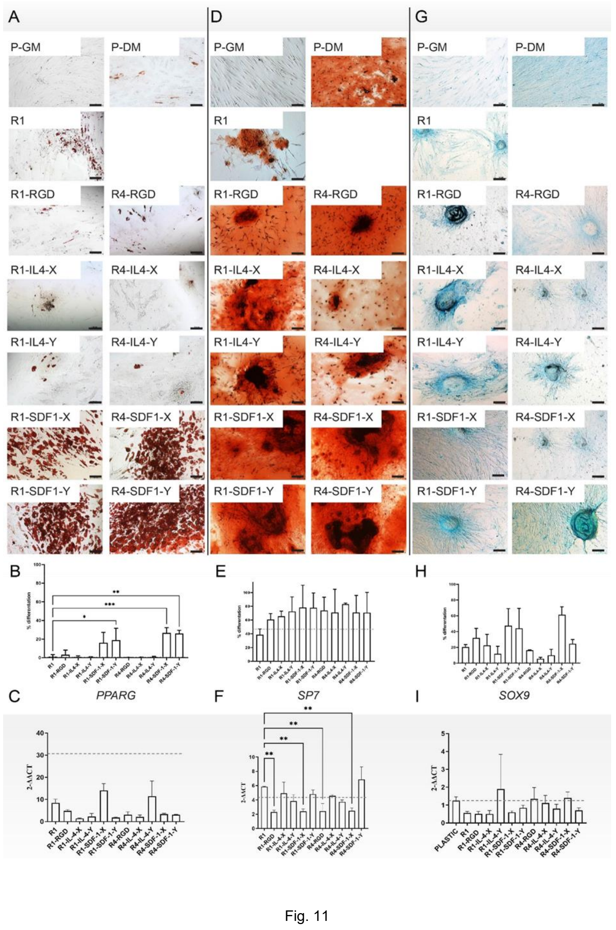

- Fig. 11.: illustrates adipogenic, osteogenic, and chondrogenic differentiation of hDFs on hydrogels. A. Representative images of hDFs after 12-14 days in adipogenic medium on hydrogels (Oil Red O staining). B. Quantification of adipogenic differentiation (differentiation area / total area); gray line indicates percentage on plastic. C. qRT-PCR analysis of PPARγ mRNA in hDFs after 12-14 days in adipogenic medium on hydrogels vs plastic. D. Representative images of hDFs after 7-14 days in osteogenic medium on hydrogels (Alizarin Red S staining). E. Quantification of osteogenic differentiation; gray line indicates percentage on plastic. F. qRT-PCR analysis of SP7 mRNA in hDFs after 7-14 days in osteogenic medium on hydrogels (data shown as 2^-ΔΔCt). G. Representative images of hDFs after 21 days in chondrogenic medium on hydrogels (Alcian Blue staining). H. Quantification of chondrogenic differentiation; gray line indicates percentage on plastic. I. qRT-PCR analysis of SOX9 mRNA in hDFs after 21 days in chondrogenic medium on hydrogels. *p ≤ 0.05, ** p ≤ 0.01, *** p ≤ 0.001.

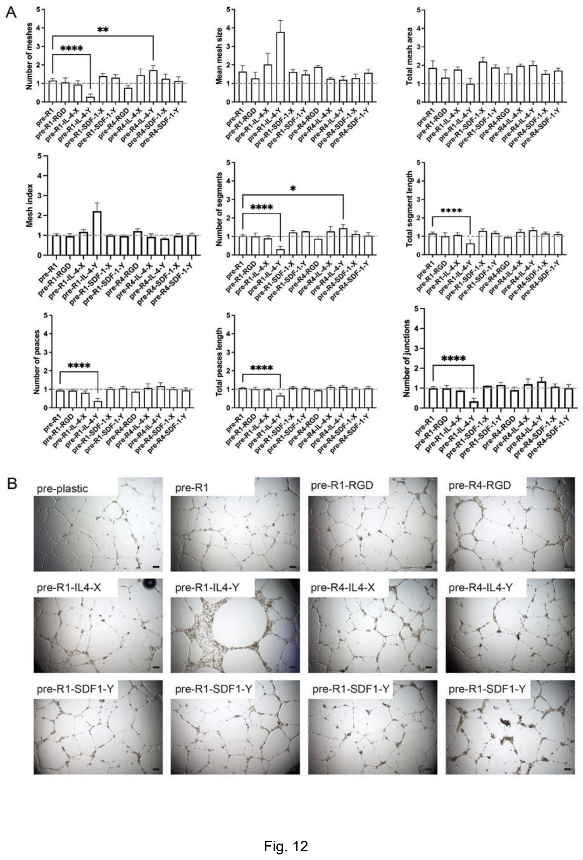

- Fig. 12.: illustrates results of HUVEC angiogenesis assay. A. Quantitative analysis of HUVEC network formation after preconditioning on hydrogels. Parameters measured include number of junctions, number of segments, total segments length, number of meshes, total mesh area, mesh index, mean mesh size. B. Representative images of capillary-like tube networks formed by HUVECs (preconditioned on hydrogels) after 18 h on Matrigel; *p ≤ 0.05, ** p ≤ 0.01, **** p ≤ 0.0001.

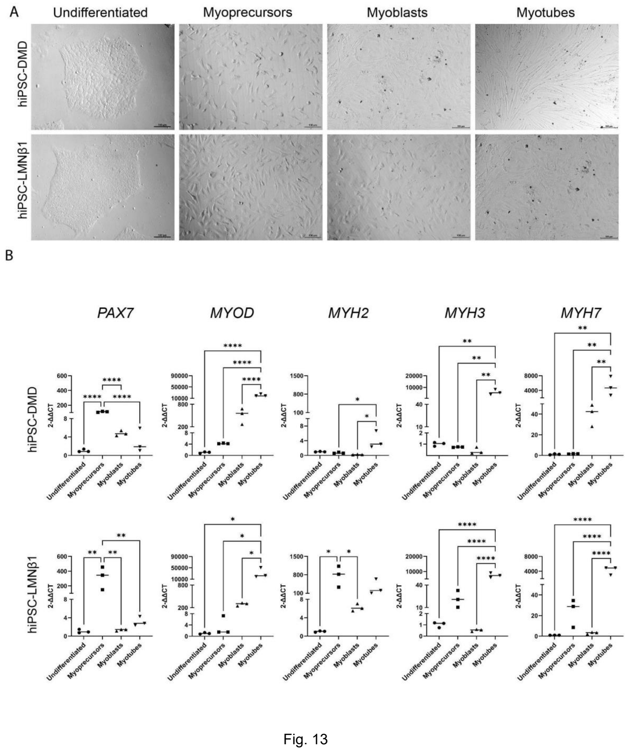

- Fig. 13.: illustrates myogenic differentiation of hiPSCs-DMD and hiPSCs-LMNβ1. A. Representative microscope images of the subsequent stages of hiPSCs myogenic differentiation analysed: undifferentiated hiPSCs, myoprecursors, myoblasts, and myotubes. Scale bar - 100 µm; B. Expression of selected myogenic markers in cells at the subsequent stages of iPSCs myogenic differentiation: undifferentiated hiPSCs, myoprecursors, myoblasts, and myotubes. The mean values of RQ (2-ΔΔCT) values with the standard deviation from at least 3 experiments are shown; undifferentiated iPSCs served as a reference sample. * p ≤ 0.05, ** p ≤ 0.01, **** p ≤ 0.0001.

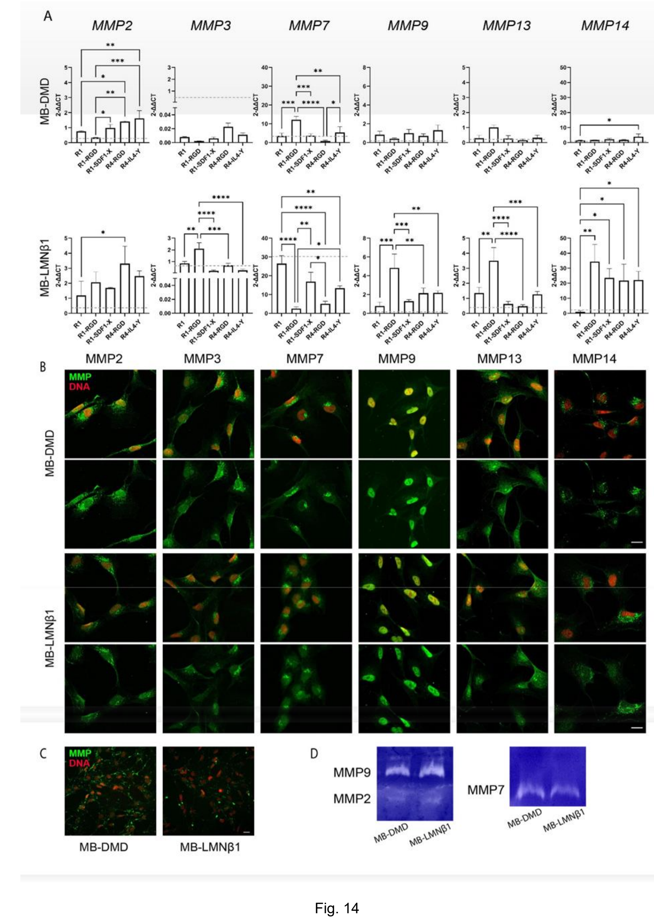

- Fig. 14.: illustrates expression and activity of selected MMPs in hiPSCs-derived myoblasts cultured on different hydrogels. A. Expression of MMP2, MMP3, MMP7, MMP9, MMP13, MMP14 in MB-DMD and MB-LMNβ1 after 3 days on hydrogels (R, R1-RGD, SEQ ID NO: 1 (R1-SDF-1-X), R4-RGD, SEQ ID NO: 8 (R4-IL-4-Y). Mean RQ (2^-ΔΔCt) ± SD (n ≥ 3). Myoblasts on day 0 served as reference. Gray line indicates expression level in collagen I control. B. Immunolocalization of indicated MMPs proteins in MB-DMD and MB-LMNβ1 cultured on gelatin-coated coverslips. Nuclei = red, MMPs = green; scale bar = 40 µm. C. In situ zymography showing gelatinolytic activity in MB-DMD and MB-LMNβ1 on day 3. Nuclei = red, activity = green. D. In-gel zymography detecting the activity of MMP-2, MMP-7, and MMP-9 in medium collected after 3 days of MB-DMD and MB-LMNβ1 culture on collagen I. *p ≤ 0.05, ** p ≤ 0.01, *** p ≤ 0.001, *** p ≤ 0.0001.

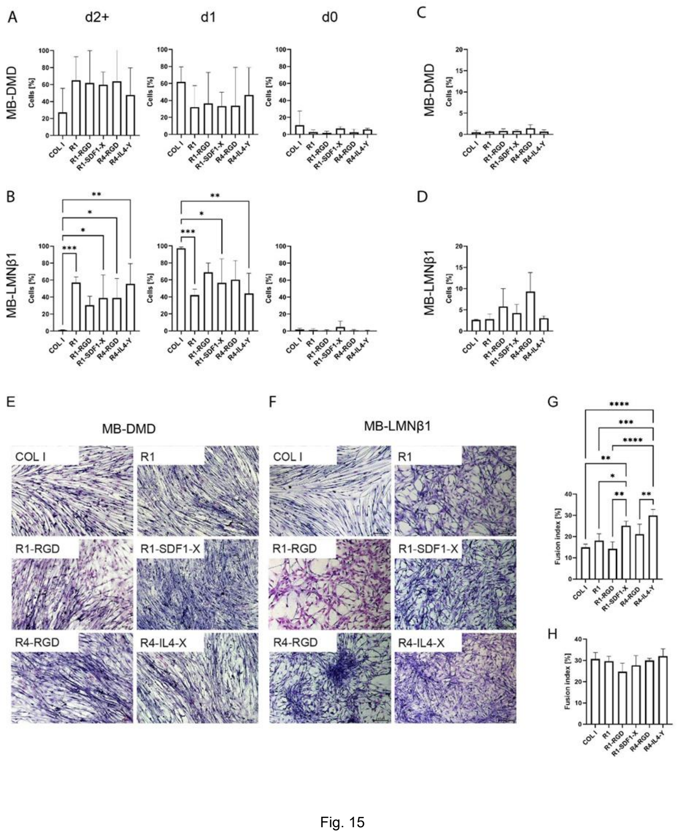

- Fig. 15.: illustrates proliferation, apoptosis, and myogenic differentiation of hiPSCs-derived myoblasts on different hydrogels. A. Percentage of MB-DMD cells undergoing two or more divisions (d2+), one (d1), or none (d0) in 48 h on indicated hydrogels or on collagen I (COL I, control). Data are mean ± SD. B. Percentage of MB-LMNβ1 cells with d2+, d1, or d0 divisions in 48 h on indicated hydrogels vs collagen I. C. Percentage of apoptotic MB- DMD cells after 72 h on indicated hydrogels vs collagen I. D. Percentage of apoptotic MB-LMNβ1 cells after 72 h on indicated surfaces. E. Representative photos of myotubes formed by MB-DMD on indicated surfaces (Giemsa-May Grünwald; scale bar = 100 µm). F. Representative photos of myotubes formed by MB-LMNβ1 on indicated surfaces (scale bar = 100 µm). G. Fusion index of MB-DMD on indicated surfaces. H. Fusion index of MB-LMNβ1 on indicated surfaces. *p ≤ 0.05, ** p ≤ 0.01, *** p ≤ 0.001, **** p ≤ 0.0001.

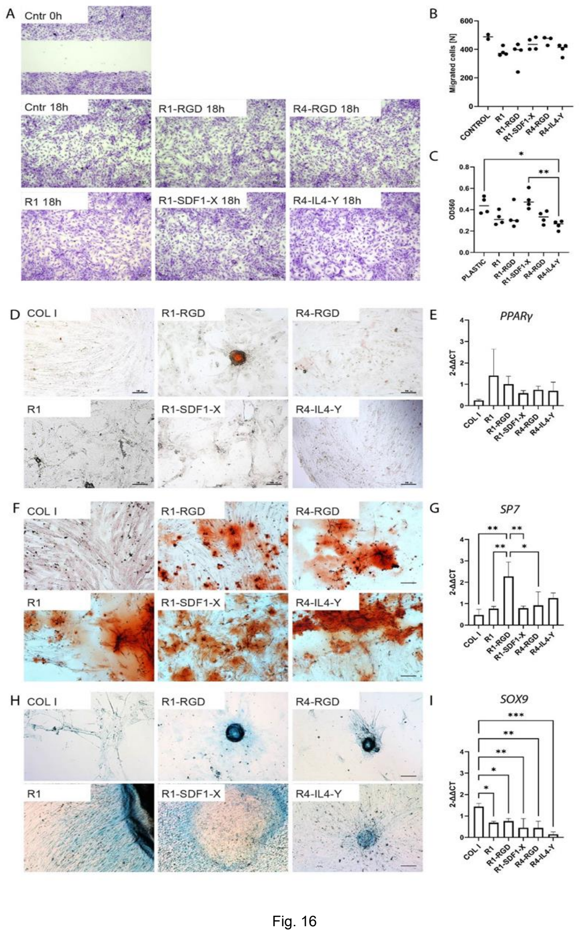

- Fig. 16.: illustrates results of assessment of migration and differentiation potential of hiPSCs-derived myoblasts on different hydrogels. A. Representative images of scratch wound healing assay after 18 h of MB-DMD culture on hydrogel-coated surfaces vs plastic (control). B. Quantification of scratch assay: number of cells migrated into the wound area (four replicates). C. Colorimetric transwell migration assay of MB-DMD on collagen I vs peptide-functionalized hydrogels after 18 h (OD at 560 nm; four replicates). D. Representative photos of MB-DMD after 14 days in adipogenic medium on hydrogels vs collagen I (Oil Red O staining of lipid droplets; scale bar = 100 µm). E. qRT-PCR analysis of PPARγ mRNA in MB-DMD after 14 days in adipogenic medium on different hydrogels vs collagen I. F. Representative photos of MB-DMD after 7 days in osteogenic medium on collagen I vs hydrogels (Alizarin Red S staining; scale bar = 100 µm). G. qRT-PCR analysis of SP7 mRNA in MB-DMD after 7 days in osteogenic medium on collagen I vs hydrogels. H. Representative photos of MB-DMD after 21 days in chondrogenic medium on collagen I vs hydrogels (Alcian Blue staining; scale bar = 100 µm). I. qRT-PCR analysis of SOX9 mRNA in MB-DMD after 21 days in chondrogenic medium on collagen I vs hydrogels. (In E, G, I: mean RQ ± SD, n ≥ 3; MB-DMD on collagen I served as reference sample.). *p ≤ 0.05, ** p ≤ 0.01, *** p ≤ 0.001.

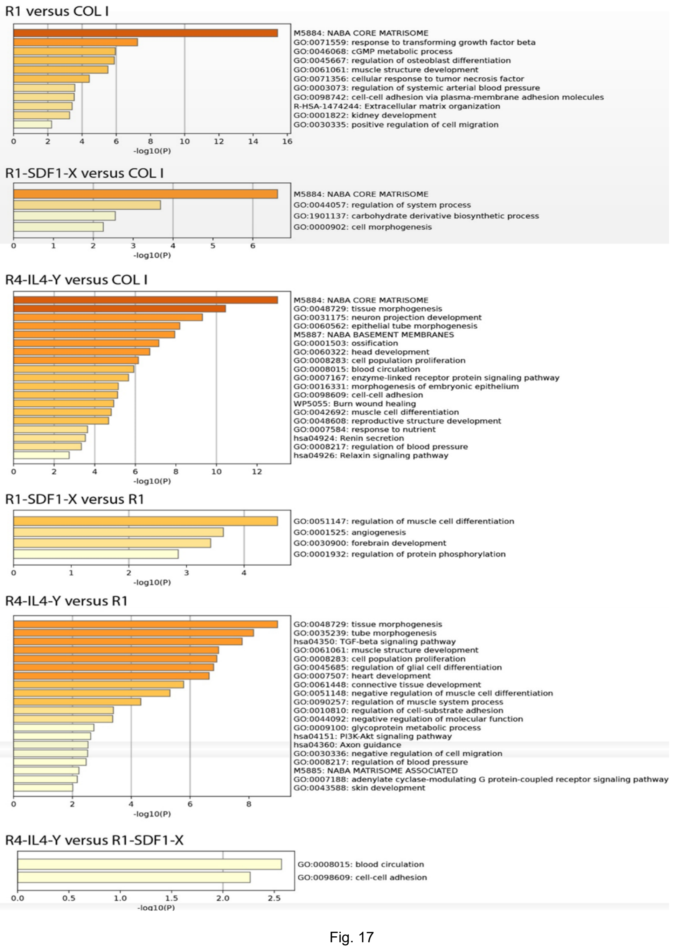

- Fig. 17.: illustrates results of gene ontology (GO) analysis of terms related to certain biological processes for transcripts most significantly up-regulated in cells cultured on different types of hydrogels. Analysis was performed with Metascape tool.

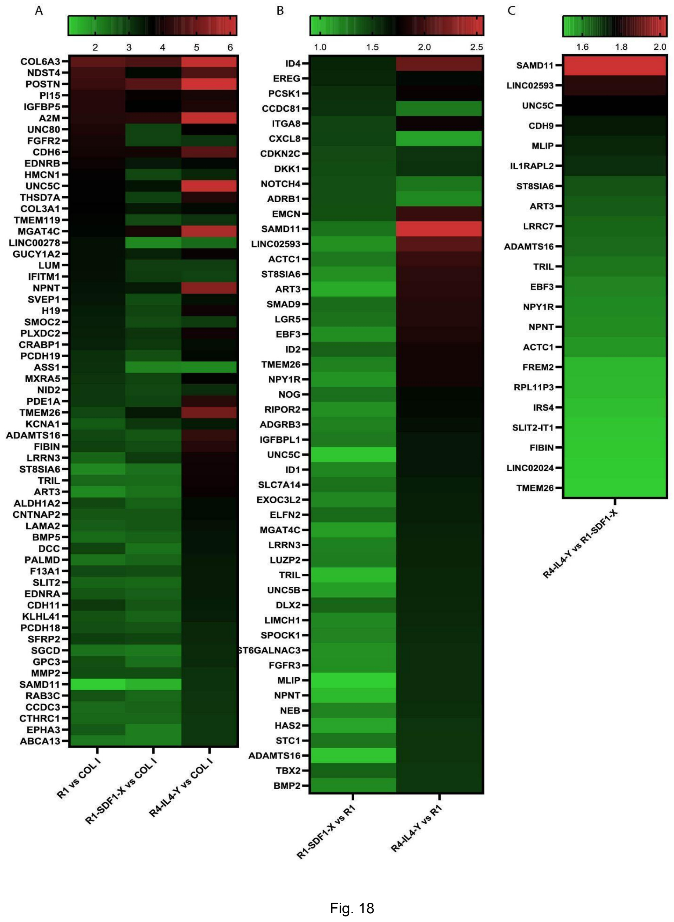

- Fig. 18.: Heat maps of transcripts up-regulated in hiPSCs-derived myoblasts cultured on different hydrogels. A. Heat map of transcripts up-regulated in myoblasts on indicated hydrogels vs collagen I (R vs Col I, SEQ ID NO: 1 (R1-SDF-1-X) vs Col I, SEQ ID NO: 8 (R4-IL-4-Y) vs Col I). The list includes transcripts with fold-change > 3 in at least one comparison. B. Heat map of transcripts up-regulated in myoblasts on functionalized hydrogels vs unmodified R (SEQ ID NO: 1 (R1-SDF-1-X) vs R, SEQ ID NO: 8 (R4-IL-4-Y) vs R). The list includes transcripts with fold-change > 1.5 in at least one comparison. C. Heat map of transcripts up-regulated in myoblasts on SEQ ID NO: 8 (R4-IL-4-Y) vs SEQ ID NO: 1 (R1-SDF-1-X) with fold-change > 1.5.

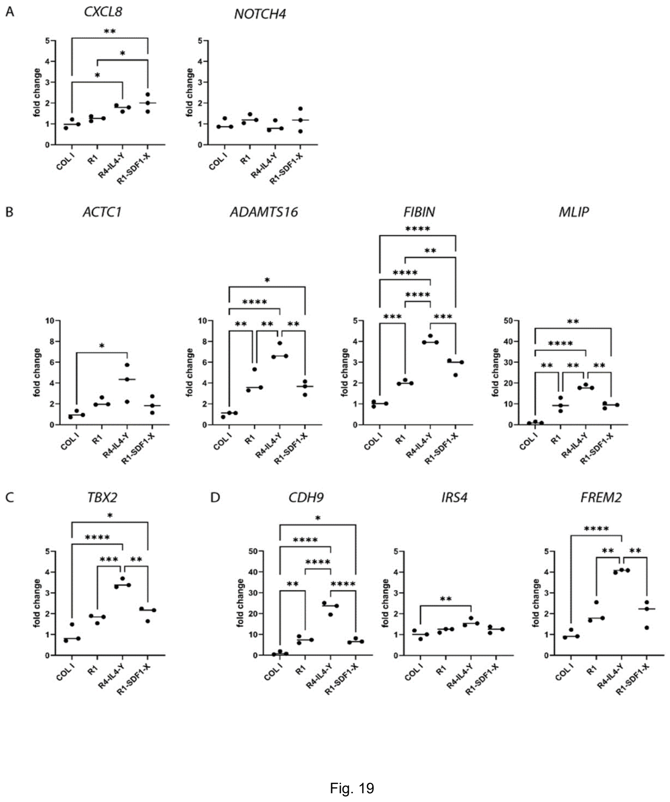

- Fig. 19.: illustrates results of the qRT-PCR analysis of transcripts selected from NGS analysis. mRNA expression was measured in MB-DMD after 3 days of culture on collagen I (control) or hydrogel-coated surfaces. Data are shown as fold-change vs collagen I. A. Transcripts found by NGS to be elevated in cells on SEQ ID NO: 1 (R1-SDF-1-X) vs R. B. Transcripts elevated in cells on SEQ ID NO: 8 (R4-IL-4-Y) vs other variants. C. Transcripts elevated on SEQ ID NO: 8 (R4-IL-4-Y) vs R. D. Transcripts elevated on SEQ ID NO: 8 (R4-IL-4-Y) vs SEQ ID NO: 1 (R1-SDF-1-X). *p ≤ 0.05, ** p ≤ 0.01, *** p ≤ 0.001, **** p ≤ 0.0001.

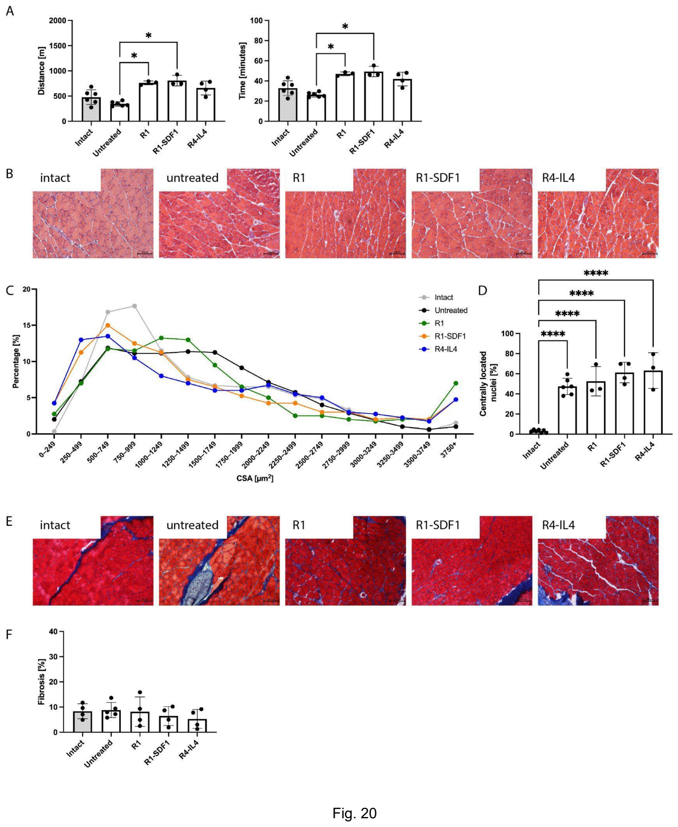

- Fig. 20.: illustrates results of treadmill test and muscle structure analysis after transplantation of hydrogels modified with IL4 or SDF1 mimicking peptides to injured SCID muscles. All analyses were performed 4 weeks after hydrogel transplantation. At least 3 samples were analyzed. A. Distance and running time during treadmill test. B. Representative images of muscle sections stained with HE; scale bar - 100 µm. C. Percentage of fibers with different CSA present in all indicated muscle groups; D. Percentage of regenerating muscle fibers with centrally located nuclei; E. Representative images of muscle sections stained with Masson's trichrome; scale bar - 100 µm; F. Fibrosis area in muscle sections (%) in all indicated muscle groups; *p ≤ 0.05, **** p ≤ 0.0001.

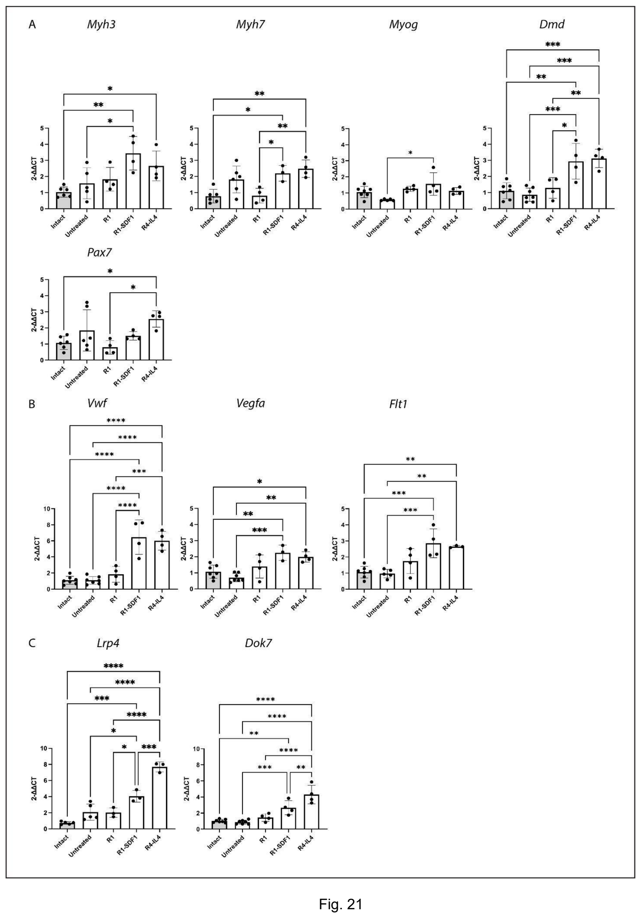

- Fig. 21.: illustrates expression of selected myogenesis, angiogenesis, and neurogenesis markers in regenerating SCID muscles injected with hydrogels modified with IL4 or SDF1 mimicking peptides. A. Myogenesis markers (Myh3, Myh7, Myog, Dmd, Pax7); B. Angiogenesis markers (Vwf, Vegfa, Flt1); C. Neurogenesis markers (Lrp4, Dok7). For A-C mean RQ (2-ΔΔCT) values with standard deviation of at least 3 experiments are shown. Intact muscles served as a reference sample. *p ≤ 0.05, ** p ≤ 0.01, *** p ≤ 0.001, **** p ≤ 0.0001.

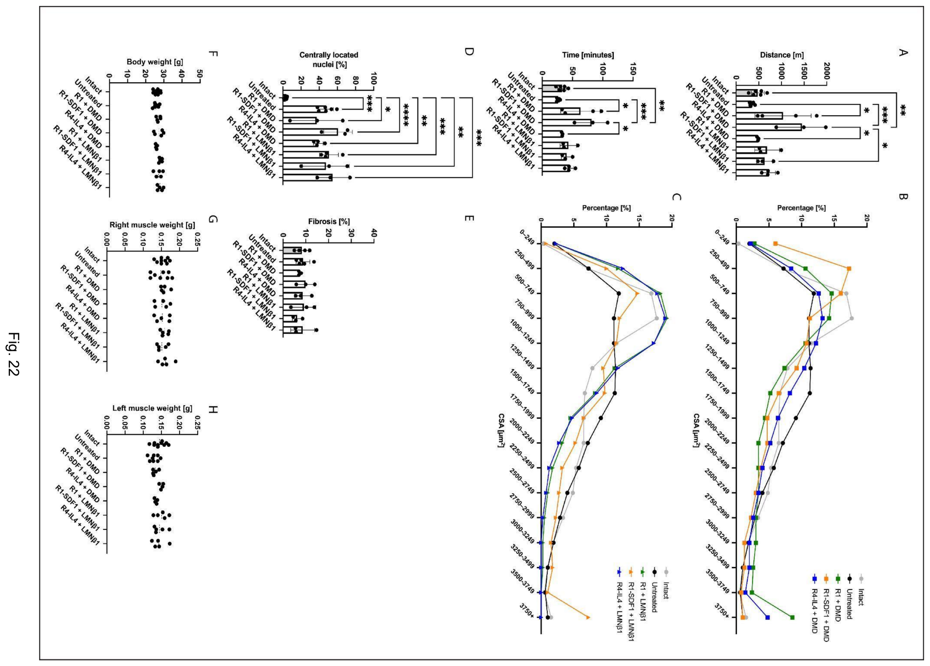

- Fig. 22.: illustrates results of treadmill test, muscle structure analysis, body and muscle weight after transplantation of hydrogels modified with IL4 or SDF1 mimicking peptides and myoblasts derived from hiPSCs to injured SCID muscles. All analyses were performed 4 weeks after transplantation of hydrogels and cells. At least 3 samples were analyzed. A. Distance and time of running during treadmill test. B. Percentage of fibers with different CSA in muscles injected with hydrogels and DMD, as well as control muscles; C. Percentage of fibers with different CSA present in muscles injected with hydrogels and LMNβ1 as well as control muscles. D. Percentage of regenerating muscle fibers with centrally located nuclei; E. Area of fibrosis in muscle sections (%) in all indicated groups; F. Weight of mice; G. Weight of muscles injured and injected with hydrogels and cells as well as weight of control muscles (intact or injured and untreated); H. Weight of contralateral muscles in all analyzed groups. *p ≤ 0.05, ** p ≤ 0.01, *** p ≤ 0.001, *** p ≤ 0.0001.

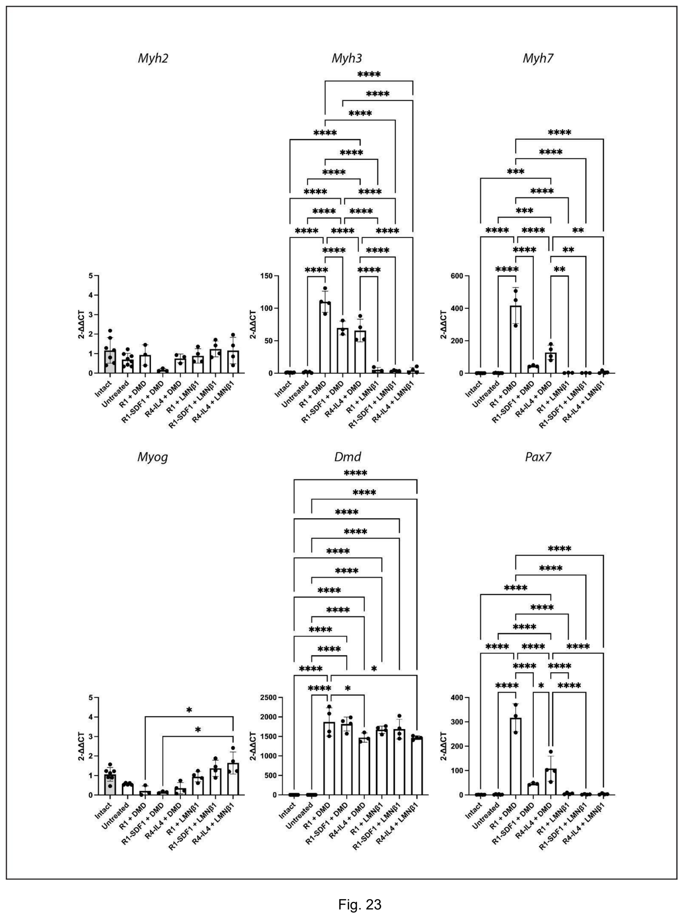

- Fig. 23.: illustrates expression of selected myogenesis markers in regenerating SCID muscles injected with hydrogels modified with IL4 or SDF1 mimicking peptides and hiPSCs-derived myoblasts. Mean RQ (2-ΔΔCT) values with standard deviation from at least 3 experiments are shown for selected myogenesis markers (Myh2, Myh3, Myh7, Myog, Dmd, Pax7). Intact muscles served as a reference sample. *p ≤ 0.05, *** p ≤ 0.0001, **** p ≤ 0.0001.

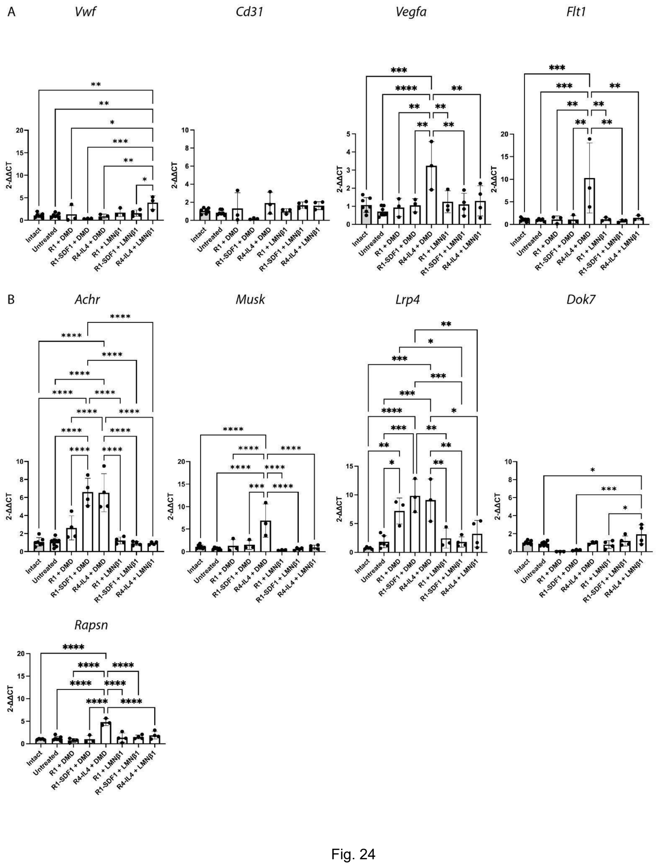

- Fig. 24.: illustrates expression of selected markers of angiogenesis and neurogenesis in regenerating SCID muscles injected with hydrogels modified with IL4 or SDF1 mimicking peptides and myoblasts derived from hiPSCs. A. Angiogenesis markers (Vwf, Cd31, Vegfa, Flt1); B. Neurogenesis markers (Achr, Musk, Lrp4, Dok7, Rapsn). Mean RQ (2-ΔΔCT) values with standard deviation from at least 3 experiments are shown. Intact muscles served as a reference sample. *p ≤ 0.05, ** p ≤ 0.01, *** p ≤ 0.001, **** p ≤ 0.0001.

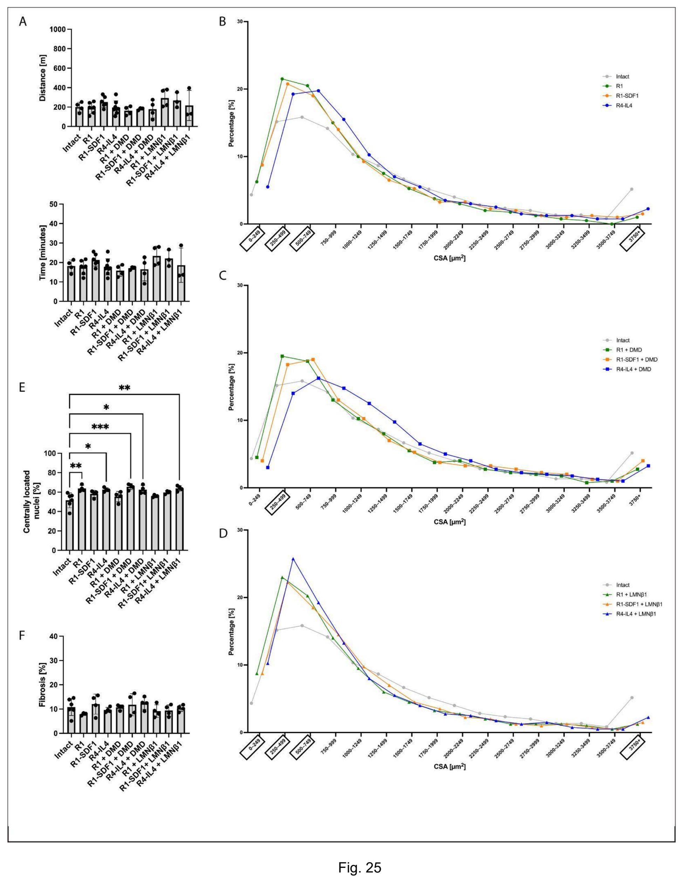

- Fig. 25.: illustrates results of treadmill test and muscle structure analysis after transplantation of hydrogels modified with IL4 or SDF1 mimicking peptides with or without hiPSCs-derived myoblasts to dystrophic muscles of SCID/mdx mice. All analyses were performed 4 weeks after hydrogel transplantation. At least 3 samples were analyzed. A. Distance and time of running during treadmill test; B. Percentage of fibers with different CSA in SCID/mdx muscles injected with hydrogels as well as control muscles; boxes indicate the fiber area category in which significant differences were found between muscle groups; C. Percentage of fibers with different CSA in SCID/mdx muscles injected with hydrogels and DMD, as in well as control muscles; D. Percentage of fibers with different CSA in SCID/mdx muscles injected with hydrogels and LMNβ1 as well as control muscles. E. Percentage of regenerating muscle fibers with centrally located nuclei. F. Area of fibrosis in muscle sections (%) in all indicated groups. *p ≤ 0.05, ** p ≤ 0.01, *** p ≤ 0.001.

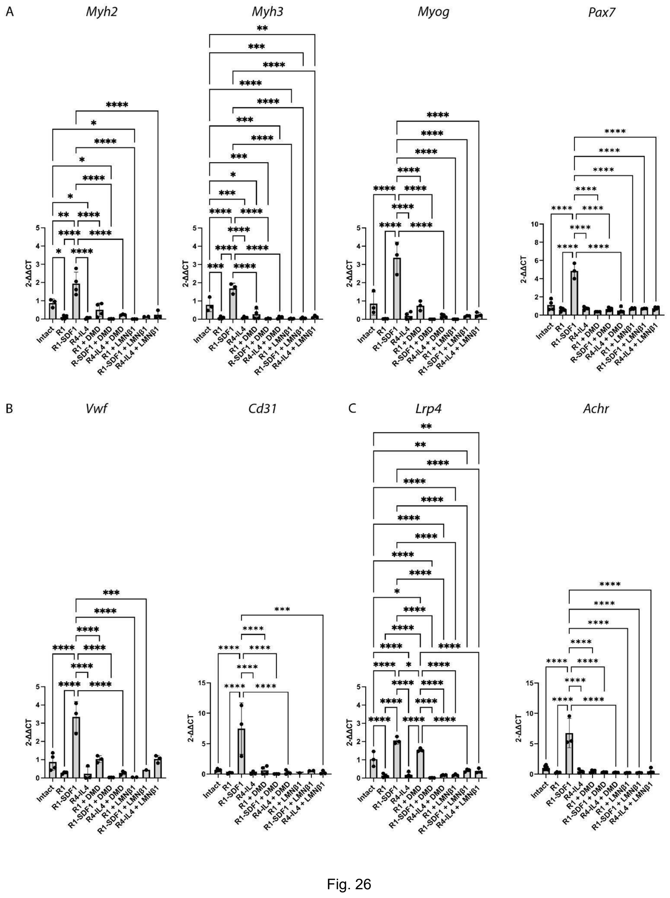

- Fig. 26.: illustrates expression of selected myogenesis, angiogenesis, and neurogenesis markers in SCID/mdx muscles after transplantation of hydrogels modified with IL4 or SDF1 mimicking peptides with or without hiPSCs-derived myoblasts. A. Myogenesis markers (Myh2, Myh3, Myog, Pax7); B. Angiogenesis markers (Vwf, Cd31); C. Neurogenesis markers (Lrp4, Achr). For A-C mean RQ (2-ΔΔCT) values with standard deviation from at least 3 experiments are shown. Intact muscles served as a reference sample. *p ≤ 0.05, ** p ≤ 0.01, *** p ≤ 0.001, **** p ≤ 0.0001.

### Detailed Description of the Invention

Extensive experiments have been conducted to demonstrate the functionality and advantages of these peptide hydrogels, both *in vitro,* in the context of skeletal muscle regeneration. In particular, the inventors evaluated the hydrogels with regard to:
a) Effects on key muscle-regenerative cell types in vitro: The influence of IL-4 and SDF-1 functionalized RADA16-I hydrogels on various human cell populations critical for muscle repair was tested. This included human skeletal myoblasts (hSkMs) (muscle precursor cells), human dermal fibroblasts (hDFs) (as a model for muscle fibroblasts contributing to extracellular matrix formation), and human umbilical vein endothelial cells (HUVECs) (representing endothelial cells involved in revascularization). Cell behaviour on the hydrogels was comprehensively analyzed, focusing on cell survival (apoptosis rate), proliferation, migratory capacity, and differentiation status, as well as changes in gene expression profiles under these culture conditions.
b) Impact on induced pluripotent stem cell-derived myoblasts: Human myoblasts derived from induced pluripotent stem cells (hiPSCs-MBs) were used to assess the hydrogels' effects on a therapeutically relevant cell source. hiPSCs provide a renewable source of patient-specific myogenic cells for potential transplantation therapies. The hydrogels were tested for their ability to support hiPSCs-derived myoblast proliferation, viability, migration, and differentiation in vitro, and whether pre-culturing ("preconditioning") these cells on the hydrogels alters their myogenic potential or gene expression (transcriptome/secretome) in beneficial ways. This aspect is significant for developing a combination therapy wherein cells are delivered together with the hydrogel.
c) Therapeutic use in treatment of muscle regeneration: The utility of the peptide hydrogels was demonstrated by transplanting them into animal models of muscle injury and disease. Experiments were conducted in immunodeficient mouse models of acute muscle injury (cardiotoxin-induced muscle damage in SCID mice) and chronic muscle degeneration (SCID/mdx mice, a model for Duchenne muscular dystrophy). The hydrogels were injected either alone or in combination with human myoblasts (including hiPSCs-derived myoblasts), and the effects on muscle regeneration and function were evaluated over time. Outcome measures included histological analysis of muscle repair (muscle fiber regeneration, fiber cross-sectional area, centrally nucleated fibers, fibrosis), functional recovery via treadmill exercise tests, and expression of genes engaged in myogenesis, angiogenesis, and innervation in the treated muscles.

The results of these investigations demonstrate that the IL-4 and SDF-1 functionalized hydrogels provide a pro-regenerative microenvironment that influences multiple cell types synergistically to promote muscle healing. Key findings from the in vitro studies on primary human cells are summarized below:
a) MMPs expression by cells on hydrogels: Crucially, the studies confirmed that the target cell populations do express the matrix metalloproteinases needed to cleave the hydrogel linkers. Analysis of MMPs gene expression on day 3 of culture showed that human myoblasts (hSkMs) express MMP-2, MMP-3, MMP-7, MMP-9, MMP-13, and MMP-14 at the mRNA level (Figure 6A-B). Fibroblasts and HUVECs similarly express multiple MMPs: for example, hDFs expressed MMP-2, -3, -7, -9, -13, -14 (Figure 7A-B); HUVECs expressed MMP-2, -3, -7, -14 (but not MMP-9 or -13) (Figure 8A-B). These enzymes were also detected at the protein level by zymography or immunostaining (e.g., myoblast and fibroblasts cultures showed active MMP-2 and MMP-9 gelatinase activity, and MMP-7 activity, during differentiation) (Figure 6C-E; Figure 7C-E). However, during HUVECs culture only MMP-7 activity was detected (Figure 8C-E). The presence of these MMPs in the local cellular environment means that, once the cells interact with the hydrogel, the peptide linkers can be cleaved, and the IL-4/SDF-1 fragments can be released to the medium culture. Indeed, some differences in MMPs expression were observed when cells were cultured on different hydrogels, suggesting the hydrogels can modulate MMPs production. For instance, fibroblasts cultured on SEQ ID NO: 7 (R4-IL-4-X) showed elevated expression of MMP3 and MMP14 relative to control hydrogel, while fibroblasts cultured on SEQ ID NO: 8 (R4-IL-4-Y) showed elevated expression of MMP3 and MMP7 compared to cell cultured on control R hydrogel. Moreover, HUVECs cultured on SEQ ID NO: 7 (R4-IL-4-X), SEQ ID NO: 5 (R4-SDF-1-X), SEQ ID NO: 6 (R4-SDF-1-Y) showed increased MMP2 or MMP14 expression compared to cells cultured on R. Such feedback implies that an IL-4 or SDF-1 hydrogel may induce cells to produce more of the very enzymes that trigger peptide release - a positive feedback loop that could accelerate the delivery of regenerative peptides when and where they are needed.
b) Cell apoptosis assay: All tested hydrogels were non-cytotoxic, supporting cell viability in culture. The percentage of apoptotic cells on peptide-modified hydrogels was generally comparable to that on the unmodified RADA16-I hydrogel (R) or standard tissue culture plastic. Notably, in the case of endothelial cells (HUVECs), the presence of SDF-1 peptide in the hydrogel significantly reduced apoptosis: HUVECs cultured on SEQ ID NO: 1 (R1-SDF-1-X) or SEQ ID NO: 5 (R4-SDF-1-X) hydrogels had a markedly lower apoptotic fraction (only ~15% apoptotic cells) compared to those on the base R hydrogel (~23% apoptotic). In other words, SEQ ID NO: 1 (R1-SDF-1-X) and SEQ ID NO: 5 (R4-SDF-1-X) hydrogels improved endothelial cell survival (Figure 9C). This indicates that SDF-1 functionalization provides pro-survival signals to endothelial cells, which could be beneficial for angiogenesis in regenerating muscle. Moreover, hydrogels did not induce excessive apoptosis during myoblasts and fibroblasts. Overall, none of the IL-4 or SDF-1 hydrogels caused increased cell death - confirming their biocompatibility in vitro.
c) Cell proliferation: The hydrogels were observed to support or enhance the proliferation of certain cell types. In particular, human fibroblasts (hDFs) responded to the modified hydrogels with increased proliferative activity. Using a CFSE dilution assay (Figure 9D-F), it was found that hDFs cultured on all IL-4 or SDF-1 peptide-modified hydrogels (except SEQ ID NO: 3 (R1-IL-4-X) hydrogel), showed a higher proportion of dividing cells (cells undergoing two or more divisions) compared to those on the unmodified R hydrogel. The myoblasts (hSkMs) and endothelial cells (HUVECs) maintained normal proliferation rates on the hydrogels (similar to R), with no deleterious effect of the peptide modifications. Thus, the presence of IL-4 or SDF-1 peptides did not inhibit cell growth; in the case of fibroblasts, it even had a stimulatory effect. This is relevant in treatment of muscle regeneration, as a moderate promotion of fibroblast proliferation by the hydrogel may aid in building an extracellular matrix for muscle repair (though unchecked fibroblast growth could also risk fibrosis, which was monitored as described later).
d) Cell migration: The ability of the hydrogels to influence myoblast migration was tested using a Transwell migration assay (where myoblast migrated through hydrogel-coated insert and were present at the membrane surface facing the bottom of the culture plate). SDF-1-functionalized hydrogels markedly enhanced the migration of myoblasts. Specifically, all hydrogels presenting SDF-1 peptides (SEQ ID NO: 1 (R1-SDF-1-X), SEQ ID NO: 2 (R1-SDF-1-Y), SEQ ID NO: 5 (R4-SDF-1-X), SEQ ID NO: 5 (R4-SDF-1-Y)) induced a greater number of hSkM myoblasts to migrate compared to control conditions without hydrogel. This is consistent with SDF-1's known chemoattractant effect on muscle progenitor cells. In contrast, an IL-4-presenting hydrogel (particularly the SEQ ID NO: 3 (R1-IL-4-X) formulation) reduced myoblast migration in this assay, as fewer cells migrated in its presence. Figure 10A illustrates these findings: SDF-1 containing hydrogels significantly increased myoblast migration relative to no-hydrogel controls (dash line), whereas SEQ ID NO: 3 (R1-IL-4-X) showed an inhibitory effect on migration (possibly because IL-4 signals favor myoblast fusion over migration, causing cells to stay and fuse rather than move). These results indicate that SDF-1 hydrogels can recruit myoblasts, an important feature for regenerating muscle, while IL-4 hydrogels may be better applied when cell fusion at a target site is desired rather than long-range cell recruitment.
e) Myogenic differentiation on hydrogels: It was important to confirm that myoblasts can still undergo normal differentiation into myotubes when cultured on the hydrogels. The studies showed that human myoblasts were indeed capable of fusing and forming multinucleated myotubes on all IL-4 and SDF-1 modified hydrogels, under differentiation conditions. The fusion index (percentage of nuclei in multinucleated myotubes) on day 6 of differentiation was slightly lower for cells on hydrogels (of any type) compared to standard tissue-culture plastic, indicating that the 3D hydrogel environment can modestly slow the differentiation rate. However, critically, there was no significant difference in myogenic differentiation efficiency between cells cultured on the peptide-functionalized hydrogels and those on the base RADA16-I hydrogel (R). In other words, adding IL-4 or SDF-1 peptides to RADA16 did not impair the myoblasts' ability to differentiate relative. Gene expression analysis of myogenic markers revealed only minor variations: for instance, at an early stage (day 3 of culture), myogenin (MYOG, a myogenic regulatory factor) mRNA was upregulated in myoblasts on all SDF-1 containing hydrogels compared to controls, suggesting a possible enhancement of differentiation signalling by SDF-1. At later stages (day 11), some hydrogels showed slightly lower levels of MYOG or certain myosin heavy chain isoforms (e.g. MYH3) in the cells, particularly SEQ ID NO: 3 (R1-IL-4-X) and SEQ ID NO: 4 (R1-IL-4-Y) in case of MYOG expression and SEQ ID NO: 4 (R1-IL-4-Y) and SEQ ID NO: 2 (R1-SDF-1-Y) hydrogels in case of MYH3 expression, but these differences did not translate into significant functional deficits in differentiation (Figure 10B-D). Overall, the conclusion was that human myoblasts can successfully undergo myogenic differentiation on the IL-4/SDF-1 hydrogels to form new muscle fibers, with no major impediment to differentiation efficiency. The peptide hydrogels thus provide a supportive surface for muscle cell fusion and maturation, while slightly modulating the expression of myogenic regulatory factors (which could potentially influence the timing of differentiation in a beneficial way).
f) Effects on fibroblast differentiation (osteogenic/adipogenic/chondrogenic potential): Muscle fibroblasts can contribute to either regenerative or pathological outcomes (fibrosis, fat infiltration, or even ossification). The behavior of hDFs on the hydrogels was examined under differentiation conditions to see if the IL-4 or SDF-1 signals push fibroblasts toward any particular lineage. The results indicated that SDF-1-presenting hydrogels may beneficially modulate fibroblast differentiation. When cultured in an adipogenic induction medium on SEQ ID NO: 2 (R1-SDF-1-Y), SEQ ID NO: 5 (R4-SDF-1-X) or SEQ ID NO: 6 (R4-SDF-1-Y) hydrogels hDFs formed slightly more lipid-laden adipocytes (fat cells) compared to those on R, although adipogenic conversion was low overall (a few percent at most). However, hDFs cultured on all IL-4/SDF-1 modified hydrogel in presence of osteogenic and chondrogenic induction medium did not show any higher level of differentiation area compared to hDFs cultured on R hydrogel. More importantly, in osteogenic induction conditions, fibroblasts on SEQ ID NO: 1 (R1-SDF-1-X) and SEQ ID NO: 5 (R4-SDF-1-X) showed significantly reduced expression of SP7, a key transcription factor for osteoblast differentiation, compared to fibroblasts on the R hydrogel (Figure 11A-I). This is a favorable outcome, as lower osteogenic differentiation of fibroblasts could mean less risk of heterotopic bone formation in regenerating muscle. Taken together, chondrogenesis and osteogenesis of fibroblasts were not promoted by the IL-4/SDF-1 hydrogels, and the SEQ ID NO: 1 (R1-SDF-1-X) and SEQ ID NO: 5 (R4-SDF-1-X) hydrogels in particular tended to keep fibroblasts away from the osteogenic fate.
g) Effects on endothelial cells (angiogenesis potential): The hydrogels' influence on endothelial cells was assessed via a tube formation assay on Matrigel after "preconditioning" the HUVECs on hydrogels (Figure 12). In general, HUVECs formed capillary-like networks after being cultured on IL-4 or SDF-1 modified hydrogels, indicating that the hydrogels did not prevent angiogenic differentiation. However, one formulation, SEQ ID NO: 4 (R1-IL-4-Y), showed a slight inhibitory effect: HUVECs preconditioned on SEQ ID NO: 4 (R1-IL-4-Y) formed fewer or shorter tubules on Matrigel compared to those preconditioned on the control R hydrogel. In contrast, SEQ ID NO: 8 (R4-IL-4-Y) hydrogel increases angiogenesis efficiency. HUVEC preconditioned with SEQ ID NO: 8 (R4-IL-4-Y) hydrogel formed more tubes with higher number of segments. Consistently, the summary of results noted that SEQ ID NO: 4 (R1-IL-4-Y) had a negative impact on HUVEC tubule formation, whereas SEQ ID NO: 8 (R4-IL-4-Y) promotes this process. Nonetheless, *all* hydrogel variants were non-toxic to endothelial cells, and aside from the specific SEQ ID NO: 4 (R1-IL-4-Y) case, endothelial network formation was largely comparable across other groups.

Overall, the in vitro findings demonstrate that all three critical cell types (myoblasts, fibroblasts, and endothelial cells) can thrive on these peptide hydrogels and that the hydrogels provide signals that collectively favor muscle regeneration: they promote myoblasts migration (SDF-1 effect) and readiness for fusion (IL-4 and SDF-1 effect), enhance fibroblasts proliferation (IL-4 and SDF-1 effect), reduce osteogenic marker expression (potentially mitigating fibrosis or ossification - SDF-1 effect), and support endothelial cell survival (SDF-1 effect) and promote angiogenesis (IL-4 effect), moreover they are all biocompatible and responsive to cellular MMPs activity.

Building on the promising in vitro results, the invention was further validated using human iPSCs-derived myoblasts (hiPSCs-MBs). Two hiPSCs cell lines were tested, that is, hiPSCs-DMD and hiPSCs-LMNβ1. The first one was characterized by the expression of dystrofin connected with green fluorescence protein (GFP), the second expression of laminin associated with GFP.
- Myogenic differentiation of hiPSCs: First, hiPSCs lines were differentiated into the myogenic lineage to generate myoblasts; this was confirmed by robust induction of muscle markers (PAX7, MYOD, MYH2, MYH3, MYH2 and MYH7, indicating the cells were bona fide myogenic precursors and myoblasts) (Figure 13). The highest level of PAX7 expression was found in the myoprecursors obtained from both cell lines. PAX7 expression was significantly elevated compared to both undifferentiated hiPSCs and more differentiated myogenic derivatives, i.e., myoblasts and myotubes. The myotubes, generated from both cell lines, were in turn characterised by the highest level of MYOD expression, significantly enhanced compared to that detected in all other samples (i.e., undifferentiated cells, myoprecursors, myoblasts). The level of mRNAs encoding all the analysed Myhs (i.e., MYH2, MYH3, MYH7) was also significantly elevated in myotubes in both cell lines, and also in myoprecursors of hiPSCs-LMNβ1. Thus, the results obtained confirmed that hiPSCs were successfully induced to undergo myogenic differentiation accompanied by expected changes in myogenic marker expression.
- MMPs expression by cells on hydrogels: In the next experiments, we investigated the expression and activity of selected types of metalloproteinases in myoblasts derived from each hiPSCs cell line (hereafter described as MB-DMD and MB-LMNβ1). MB-DMD and MB-LMNβ1 were cultured on different types of hydrogels (R, R1-RGD, SEQ ID NO: 1 (R1-SDF-1-X), R4-RGD, SEQ ID NO: 8 (R4-IL-4-Y)) or on collagen I as a control. The analysis of MMPs was a pivotal stage of this research, as our hydrogels were modified with IL4 or SDF1 mimicking peptides by linking them to the hydrogel with a sequence recognized by MMPs - PAGL or VLGL. Therefore, these enzymes are crucial to release IL4 and SDF1 mimicking peptides from hydrogels. MB-DMD cultured on the SEQ ID NO: 8 (R4-IL-4-Y) hydrogel were characterized by significantly higher expression of MMP2 and MMP14, while MB-LMNβ1 culture on the same variant showed increased in MMP7 and MMP14 expression compared to cells cultured on unmodified R hydrogel. In case of SEQ ID NO: 1 (R1-SDF-1-X) hydrogel, a significant upregulation in MMP2 (MB-DMD line), MMP7 and MMP14 (MB-LMNβ1 line) expression was observed compared to cells cultured on control R or R1-RGD hydrogel (Figure 14A). To verify whether MMPs proteins are present and active in hiPSCs-derived myoblasts, RT-qPCR analysis was followed by immunocytochemistry and zymography in situ. All analyzed MMPs were successfully detected by immunocytochemistry in both MB-DMD and MB-LMNβ1 (Figure 14B). The activity of MMP2, MMP7, and MMP9 was also shown using zymography (Figure 14C-D). In summary, all MMPs required for peptide release from the hydrogel backbone were detected at both the transcript and protein level in myoblasts obtained from both lines of hiPSCs. Furthermore, the presence of custom-designed hydrogels modified with IL4 and SDF1 mimicking peptides had a noticeable impact on the expression of some analyzed MMPs in hiPSCs-derived myoblasts, as shown by comparison with controls.
- Cell proliferation and apoptosis: Next, we investigate the impact of custom-designed hydrogels modified with IL4 or SDF1 mimicking peptides on proliferation and apoptosis of MB-DMD and MB-LMNβ1. For proliferation assessment, CFSE assay was used, which revealed no significant differences between MB-DMD, cultured on collagen I or on different types of hydrogels. In case of MB-LMNβ1 we found that a significantly higher proportion of cells cultured on R or SEQ ID NO: 1 (R1-SDF-1-X) or SEQ ID NO: 8 (R4-IL-4-Y) underwent two or more divisions compared to control cells cultured on collagen I and, at the same time, a lower proportion of cells after one division was found in these samples. Furthermore, a higher proportion of cells cultured on the R4-RGD hydrogel underwent two or more divisions compared to control cells. Thus, more MB-LMNβ1 cultured on indicated hydrogel types (R, SEQ ID NO: 1 (R1-SDF-1-X), R4-RGD, SEQ ID NO: 8 (R4-IL-4-Y)) underwent 2 or more divisions than cells cultured on collagen I as well as on R1-RGD hydrogel (Figure 15A-B). Next, we evaluated the impact of the hydrogels tested on cell survival by assessing the proportion of cells undergoing apoptosis and found - for both analyzed cell lines - no significant differences between all samples, including control cells cultured on collagen I (Figure 15C-D).
- Myogenic differentiation on hydrogels: Subsequently, we focused on the influence of hydrogels on the differentiation of myoblasts cultured on them. MB-DMD and MB-LMNβ1 were cultured for 3 days in a proliferating medium followed by a 6-day culture in myotube induction medium. The cells were fixed, stained, and the fusion index was determined, that is, the proportion of nuclei in myotubes to all nuclei. Importantly, myoblasts underwent successful myogenic differentiation manifested by myotube formation when cultured on any type of hydrogel tested as well as on collagen I. In the case of MB-DMD significantly more effective differentiation occurred when they were cultured on SEQ ID NO: 1 (R1-SDF-1-X) or SEQ ID NO: 8 (R4-IL-4-Y) hydrogels compared to cells cultured on collagen I as well as R, R1-RGD and R4-RGD (for cells cultured on SEQ ID NO: 8 (R4-IL-4-Y)). Thus, modification of hydrogels with SDF1 or IL4 mimicking peptides clearly promoted myogenic differentiation of MB-DMD. No significant differences in the values of the fusion index were found for MB-LMNβ cells (Figure 11E-H).
- Cell migration: Because the ability to migrate is an essential feature of functional myogenic cells as well as crucial for effective cell therapy designed for skeletal muscles, in the next experiments we investigated this property in MB-DMD cultured on tested hydrogels or on plastic. In these experiments we used only one type of myoblasts, i.e. MB-DMD, as these cells were characterized by better myogenic differentiation compared to MB-LMNβ1. Scratch assay revealed that cells cultured on all analyzed hydrogels as well as on collagen I were able to migrate. No significant differences were found between them (Figure 16A-B). However, using a colorimetric cell migration assay, we found that MB-DMD cultured on a SEQ ID NO: 8 (R4-IL-4-Y) hydrogel migrated less efficiently than those cultured on R1-SDF1 X or on plastic, but comparable to all other variants of the experiment (Figure 16C).
- Effects on hiPSCs-derived myoblasts osteogenic, adipogenic and chondrogenic differentiation potential: we verified whether hiPSCs-derived myoblasts underwent differentiation other than myogenic, i.e., adipogenic, osteogenic or chondrogenic. Oil Red O stained virtually any or only single cells that originated from MB-DMD cultured on indicated hydrogels or collagen I, in a medium promoting adipogenesis. Also, the level of PPARγ, adipogenic marker, was low and similar in all samples analyzed (Figure 16D-E). The osteogenesis assessed by Alizarin Red S staining was similar for all variants of the experiments, except for MB-DMD cultured in collagen I, which were characterized by significantly lower levels of such differentiation. The expression of the osteogenic marker, SP7, was similar in all the samples except for cells cultured on the R1-RGD hydrogel which were characterized by significantly higher levels of this mRNA (Figure 16F-G). Finally, Alcian Blue staining revealed a limited level of chondrogenic differentiation of MB-DMD cultured on R1-RGD, R4-RGD, and SEQ ID NO: 8 (R4-IL-4-Y), as well as on collagen I. Furthermore, the expression of the chondrogenic marker, SOX9, was low in all samples analyzed. A significantly higher level of this marker was found in MB-DMD cultured on collagen I than in all other variants (Figure 16H-I).
- NGS analysis: To better assess the influence of the hydrogels tested on hiPSCs-derived myoblasts, we performed a transcriptome analysis by next generation RNA sequencing using MB-DMD cultured on collagen I, R, SEQ ID NO: 1 (R1-SDF-1-X), or SEQ ID NO: 8 (R4-IL-4-Y) hydrogels. By analyzing fold changes, we found numerous differentially expressed transcripts in MB-DMD cultured on tested surfaces. The annotations of gene ontology (GO) performed using Metascape tool revealed that significantly up-regulated transcripts in analyzed cells were associated with processes such as ECM formation (for cells cultured on all types of hydrogels tested), adhesion (cells cultured on R or SEQ ID NO: 8 (R4-IL-4-Y) hydrogels) or muscle cell development and differentiation (for cells cultured on all types of tested hydrogels) (Figure 17). As no or limited information was found for down-regulated transcripts, we further focused on those whose expression was elevated. Thus, we detailly followed the differences in transcript expression found in MB-DMD cultured on hydrogels compared to those cultured on collagen I (R vs collagen I; SEQ ID NO: 1 (R1-SDF-1-X) vs collagen I, SEQ ID NO: 8 (R4-IL-4-Y) vs collagen I) as well as between cells cultured on hydrogels modified with SDF1 or IL4 peptides in comparison to cells cultured on control hydrogel (SEQ ID NO: 1 (R1-SDF-1-X) vs R1; SEQ ID NO: 8 (R4-IL-4-Y) vs R) and SEQ ID NO: 8 (R4-IL-4-Y) vs SEQ ID NO: 1 (R1-SDF-1-X) (Figure 18). Based on the literature data we have indicated the list of 10 transcripts that were elevated in cells cultured on different types of hydrogels and which are known to play an important role and/or are involved in pathways crucial for myogenic cell development, maintenance, and differentiation. Next, the level of transcripts, revealed by NGS analysis as differentially expressed in MB-DMD cultured on different types of hydrogels, was evaluated by RT-qPCR. As indicated by the NGS results, a significantly higher level of CXCL8 was found in myoblasts cultured on the SEQ ID NO: 1 (R1-SDF-1-X) hydrogel. Furthermore, it was also enhanced in MB-DMD cultured on SEQ ID NO: 8 (R4-IL-4-Y) hydrogels compared to control cells. The second transcript selected on the NGS results, NOTCH4, was expressed at a similar level in all types of samples. Next, we found that mRNAs encoding ACTC1, ADAMTS16, FIBIN, and MLIP, i.e., genes that were indicated by NGS analysis as significantly up-regulated in cells cultured on the SEQ ID NO: 8 (R4-IL-4-Y) hydrogel compared to other experiment variants (collagen I, R1, SEQ ID NO: 1 (R1-SDF-1-X)), were indeed significantly elevated in such cells. Except for ACTC1, the significantly higher level of these mRNAs was also found in cells cultured on R or SEQ ID NO: 1 (R1-SDF-1-X) hydrogels compared to control cells propagated on collagen I. The TBX2 mRNA was also significantly elevated in cells cultured on SEQ ID NO: 8 (R4-IL-4-Y) hydrogel compared to other samples, as well as in cells propagated on SEQ ID NO: 1 (R1-SDF-1-X) hydrogel in comparison to control cells. However, cells cultured on the SEQ ID NO: 8 (R4-IL-4-Y) hydrogel were characterized by significantly higher levels of CDH9, IRS4 and FREM2 as indicated by NGS analysis (Figure 19).

The comprehensive transcriptomic and secretomic analyses suggested that preconditioning myoblasts on the IL-4/SDF-1 hydrogels can modulate the cells' gene expression in beneficial ways, which might improve their regenerative capacity upon transplantation. Thus, hydrogels can be used not only as delivery vehicles but also as ex vivo conditioning matrices for cell therapy, improving myoblast preparations before they are delivered into injured muscle.

Finally, in vivo studies in mice demonstrated the hydrogels' capacity to enhance muscle regeneration. In an acute injury model, SCID mice with cardiotoxin-induced muscle damage were treated with injections of the hydrogels alone (SEQ ID NO: 1 (R1-SDF-1-X) and SEQ ID NO: 8 (R4-IL-4-Y) described below as R1-SDF1 and R4-IL4). The outcomes were measured after 4 weeks after transplantation:
a) Functional test: It revealed that mice whose muscles were transplanted with R1-SDF1 hydrogel were characterized by the highest running distance and time. These results were significantly higher compared to injured but untreated muscles. Significant differences were also found between mice which muscles were injected with control hydrogel and mice which muscles were untreated (Figure 20A). To assess whether hydrogel transplantation influenced the structure of regenerating muscles, we stained the histological sections obtained from them with HE and then CSA and the percentage of regenerating fibers, characterized by centrally located nuclei were determined. We did not find any significant differences between all analyzed muscle groups (Figure 20B-D). Next, we analyzed muscle sections stained with Masson's trichrome to determine the level of fibrosis in all muscle types and again did not find any significant differences between them (Figure 20E-F). Altogether, the obtained results indicated that reconstruction of the injured muscle in the presence of hydrogels proceeded properly and resulted in functional muscle enhancement, as suggested by the outcome of the treadmill test.
b) Gene expression analysis: we analyzed the expression of selected myogenesis, angiogenesis, and neurogenesis markers in all muscle groups (intact, untreated, transplanted with R, R4-IL4, or R1-SDF1) to determine whether the hydrogel presence influenced muscle reconstruction, including their innervation and vascularization. As myogenic markers we analyzed three types of myosin heavy chains, i.e. Myh3, which is expressed in embryonic and fetal but also in regenerating fibers, as well as Myh2 and Myh7, which are present in more mature fast and slow muscle fibers. In addition, we analyzed Myog, which is necessary for the differentiation of myogenic cells, Dmd, which encodes DYSTROPHIN, a structural protein that protects the integrity of muscle fiber membrane, and finally Pax7, a marker of SCs, which are crucial for muscle regenerative potential. Among the angiogenesis markers analyzed by us there were Vwf, Cd31, Vegfa, and Flt1, while the neurogenesis markers included Achr, Musk, Lrp4, Dok7, and Rapsn. Obtained results indicated that muscles injected with R4-IL4 were characterized by significantly higher levels of Myh7, Dmd, and Pax7 expression compared to muscles injected with unfunctionalized hydrogel, as well as control, intact muscles. In the case of Dmd mRNA, a significant difference was also found between the muscles injected with R4-IL4 and the injured untreated ones. The level of Dmd expression was also significantly higher in muscles injected with R1-SDF1 hydrogel compared to intact and untreated muscles and muscles injected with unfunctionalized hydrogel. Such muscles, i.e., transplanted with R1-SDF1, were also characterized by significantly higher levels of Myog mRNA, compared to untreated ones, as well as Myh7 mRNA, compared to intact muscles and muscles injected with control hydrogel. In addition, both muscles injected with functionalized hydrogels were characterized by an elevated level of Myh3 expression compared to intact muscles, and in case of R1-SDF1 treated muscles - also to untreated ones. These results suggest that both groups of muscles injected with functionalized hydrogels, i.e., R1-SDF1 and R4-IL4, were characterized by enhanced level of myogenesis markers (Figure 21A). Also, in the case of mRNAs encoding angiogenesis markers, we found a significantly higher level of them in muscles transplanted with functionalized hydrogels - Vwf compared to all other muscle groups (i.e., intact, untreated, R) and Vegfa and Flt1 in comparison to intact and untreated muscles (Figure 21B). The expression of neurogenesis markers was also significantly elevated in muscles injected with functionalized hydrogels. Significantly higher levels of Dok7 and Lrp4 expression were found in such muscles compared to intact and untreated ones. In the case of R4-IL4 treated muscles, the level of Dok7 mRNA was higher than in muscles injected with other hydrogels and Lpr4 mRNA was elevated in muscles treated with functionalized hydrogels compared to those injected with R hydrogel (Figure 21C). All of the results indicate that transplantation of each functionalized hydrogel was beneficial for skeletal muscle reconstruction as it supported expression of mRNAs encoding factors indispensable for myogenesis, angiogenesis, and innervation, i.e., processes that are crucial for regaining proper muscle functioning.

Hydrogel and hiPSCs-derived myoblasts injection: In the next stage of our studies, muscles of SCID mice were injured with BaCl2 and then transplanted not only with hydrogels but also with myoblasts obtained from hiPSCs. Two lines of hiPSCs were used, i.e., hiPSCs-DMD-GFP, synthesizing DYSTROPHIN linked to the GFP protein as well as hiPSCs-LMNβ1-GFP, producing fluorescently tagged LAMINβ1. As a result, two types of myoblasts were generated - MB-DMD and MB-LMNβ1 - hereinafter called DMD and LMNβ1, respectively.
a) Functional test: 28 days after transplantation, we performed a treadmill test and found the highest running distance and time for mice which muscles were injected with R1-SDF1 hydrogel and DMD cells. Significantly elevated values were also observed in the case of mice which muscles were injected with control hydrogel and DMD cells, compared to those which muscles were untreated. Detailed analysis of muscle structure, including assessment of CSA, percentage of fibers with centrally located nuclei as well as fibrosis revealed no significant differences between all muscle groups (Figure 22).
b) Gene expression analysis: Analysis of the expression of myogenesis, angiogenesis, and neurogenesis markers showed that muscles injected with each type of hydrogel and DMD cells were characterized by significantly higher level of Myh3 expression in comparison to other variants of the experiment. All types of muscles transplanted with hydrogel and cells, regardless of their types, had a significantly elevated level of Dmd mRNA in comparison to intact and untreated muscles. Additionally, a significantly higher level of Pax7 mRNA was observed in the R and DMD injected muscles compared to all other muscle groups. Elevated Pax7 expression was also found in muscles injected with R4-IL4 and DMD. Both indicated muscle groups (i.e., injected with R or R4-IL4 hydrogel and DMD cells) were also characterized by significantly elevated level of Myh7 expression compared to other muscle groups (Figure 23). For angiogenesis markers, the most profound effect was observed for muscles injected with R4-IL4 hydrogel and DMD. These muscles were characterized by elevated expression of Vegfa and Flt1 compared to all other muscles. Furthermore, we also observed a significantly higher level of Vwf mRNA in R4-IL4 and LMNβ1 injected muscles as compared to other muscle groups (Figure 24A). The most profound effect as far as neurogenesis marker expression was concerned, we again found for the muscles injected with R4-IL4 and DMD which were characterized by a significantly higher level of Musk and Rapsn mRNAs as compared to all other muscle groups. Furthermore, such muscles, as well as muscles injected with R1-SDF and DMD, had significantly elevated level of Achr mRNA compared to all other samples. All muscle groups injected with hydrogels and DMD cells were also characterized by a higher level of Lrp4 expression comparing to control muscles as well as those injected with hydrogels and LMNβ1 cells. Furthermore, muscles injected with R4-IL4 and LMNβ1 were characterized by a significantly higher Dok7 mRNA level compared to untreated muscles as well as other selected ones (Figure 24B). Together, the results suggest that addition of hiPSCs-derived myoblasts, with properties similar to DMD myoblasts used in the current study, may support myogenesis, angiogenesis, and neurogenesis in regenerating muscles.

Dystrophic muscle model: In the next set of experiments, we transplanted hydrogels with or without myoblasts derived from hiPSCs to dystrophic muscles of SCID/mdx that serve as an animal model of Duchenne's muscular dystrophy. Dystrophic muscles which were not treated served as a control; they are further described as intact muscles
a) Functional test and histological structure: The treadmill test performed 28 days after hydrogel transplantation with or without cells (DMD or LMNβ1 myoblasts) to dystrophic muscles of SCID/mdx mice did not reveal any significant differences between all muscle groups. Detailed analysis of muscle structure and CSA revealed numerous significant differences between muscle groups, especially in the case of the smallest (CSA from 0-749 µm) and the largest fibers (CSA above 3750 µm). Histological analysis of Masson's trichrome stained muscle sections indicated that fibrosis level was similar in all analyzed muscle groups, i.e., was not enhanced by any hydrogel or hydrogel and cell transplantation (Figure 25).
b) Gene expression analysis: Analysis of myogenesis, angiogenesis, and neurogenesis markers revealed a significantly higher level of Myh2, Myh3, Myog, Pax7, Vwf, Cd31, Lrp4 and Achr expression in dystrophic muscles injected with the R1-SDF1 hydrogel. In case of Lrp4, its significantly higher expression level was also found in dystrophic muscle injected with R hydrogel and DMD cells compared to all other samples, except the already mentioned R1-SDF1 treated muscles. No other significant enhancement was observed for all other genes analyzed in dystrophic muscles treated with hydrogels with or without cells. Their expression level was similar or lower than that in intact muscles. Thus, the obtained results indicate that addition of cells to hydrogels transplanted to muscles of dystrophic SCID/mdx mice did not enhance the beneficial effect of hydrogel alone, as was observed in case of injured muscles of SCID mice. The most profound effect manifested by the enhanced expression of myogenesis, angiogenesis, and neurogenesis markers was visible for muscle transplanted with R1-SDF1 hydrogel (Figure 26). Injection of the other functionalized hydrogels alone or with cells (regardless of their type), resulted in an increased number of regenerating fibers, characterized by centrally located nuclei. Such a situation may result in improvement of muscle structure and functionality after additional time, which will be verified by us in further studies.

In conclusion, the invention provides peptide-functionalized hybrid hydrogels that act as a scaffold for muscle tissue engineering and as a delivery system for regenerative peptides in one. The experimental results verify that these hydrogels: (1) are biocompatible and do not harm cells or tissue; (2) respond to muscle-specific MMPs to release IL-4 and SDF-1 signals in a controlled way; and (3) actively enhance various aspects of muscle regeneration - including myoblast proliferation, migration and fusion, modulation of fibroblast behavior to reduce fibrosis/ossification, and support of angiogenesis and innervation - leading to improved muscle healing *in vivo.* Figures 1-13 and 18-24 of the application illustrate the design and effects of the hydrogels, with Figure 1 depicting the hydrogel's structural concept, Figure 2-5 physicochemical properties and Figures 6-16 showing cellular-level interactions (MMPs expression, cell assays), while Figures 20-24 present the in vivo functional and histological outcomes. Taken together, these results highlight that peptide hydrogels carrying IL-4 and SDF-1 fragments (particularly SEQ ID NO: 1 (R1-SDF-1-X) and R4-IL-4-Y) have significant potential as therapeutic biomaterials for skeletal muscle regeneration. This invention thus opens a promising avenue for treating muscle injuries and degenerative muscle diseases by providing an MMP-responsive hydrogel scaffold that synergistically promotes muscle fiber regeneration, reduces pathological tissue responses, and improves functional recovery.
In summary, the inventive concept resides in a peptide-based hydrogel (exemplified by formulations such as SEQ ID NO: 1 (R1-SDF-1-X) and R4-IL-4-Y) that integrates scaffold support with biochemical signaling in a temporally controlled fashion. It introduces a new therapeutic platform for muscle regeneration where the scaffold itself actively participates in the healing process by releasing fusion-promoting and chemoattractant signals in response to the regenerating tissue's enzymatic activity. This targeted, enzyme-triggered delivery of IL-4 and SDF-1 peptide cues - in combination with the intrinsic biocompatibility and tailorability of the RADA16-I hydrogel - distinguishes the invention from existing muscle repair materials and provides a foundation for more effective regenerative therapies.

The described peptide-functionalized hydrogels offer numerous advantages over unmodified scaffolds or conventional delivery methods, as evidenced by comprehensive in vitro and in vivo studies. The key benefits and unexpected results conferred by the invention are outlined below:
a) Biocompatibility and Safety: The hydrogels were shown to be highly biocompatible with relevant cell types. *In vitro,* none of the functionalized hydrogels caused cytotoxic effects - no increase in apoptosis was observed in human myogenic cells cultured on them. In fact, certain formulations actively reduced cell apoptosis; for example, hydrogels containing SDF-1 significantly decreased programmed cell death in endothelial cells (HUVECs) compared to controls. Moreover, unlike many protein-based treatments, the use of IL-4 and SDF-1 peptide fragments minimizes the risk of an adverse immune reaction. This approach significantly decreases inflammatory responses relative to hydrogels bearing whole proteins, demonstrating the material's safety and suitability for implantation.
b) Enhanced Myoblast Recruitment and Fusion: The inventive hydrogels actively promote the fundamental cellular processes of muscle regeneration. SDF-1-functionalized hydrogels were found to stimulate human myoblast migration in vitro, effectively recruiting more muscle precursor cells to the site. All hydrogels bearing SDF-1 fragments also enhanced myogenic differentiation signals (e.g. they upregulated the myogenin gene, MYOG, a marker of differentiation) in cultured myoblasts. In parallel, the IL-4 and SDF-1 peptide additives improved the fusion of hiPSCs-derived myoblasts into myotubes. Notably, hiPSCs-derived myoblast culture on IL-4- or SDF-1-functionalized hydrogels exhibited an increased fusion index, meaning a greater proportion of myoblasts successfully fused into multinucleated muscle fibers. By fostering both the migration of myogenic cells to the injury and their subsequent fusion into new muscle fibers, the hydrogel provides a significantly more effective environment for muscle tissue rebuilding than standard scaffolds.
c) Support of Regenerative Support Cells (Fibroblasts and Others): Beyond direct effects on muscle cells, the hydrogels beneficially modulate other cell populations essential for regeneration. Human dermal fibroblasts (which contribute to extracellular matrix formation in muscle) showed increased proliferation on all functionalized hydrogels, indicating that the scaffold supports the growth and activity of these supportive cells. Importantly, certain SDF-1-containing hydrogels also suppressed unwanted osteogenic differentiation in fibroblasts: the expression of an osteogenic marker (SP7) was downregulated in fibroblasts cultured on SEQ ID NO: 1 (R1-SDF-1-X) and SEQ ID NO: 5 (R4-SDF-1-X) hydrogels. This is a valuable advantage, as it suggests a reduced risk of fibroblasts differentiating abnormally into bone or calcific tissue within the muscle (heterotopic ossification is a known complication in muscle trauma). Thus, the material supports the constructive role of fibroblasts in healing (laying down matrix for muscle fibers) while mitigating pathways that could lead to fibrosis or calcification.
d) Pro-Angiogenic and Endothelial-Supportive Effects: The inventive hydrogel also creates a milieu conducive to angiogenesis, which is crucial for supplying blood to regenerating muscle. Endothelial cells remained healthy and functional in the presence of the hydrogels; for instance, SDF-1-functionalized versions significantly reduced endothelial cell apoptosis in vitro, thereby preserving viable cells for new vessel formation. *In vivo* studies further confirmed enhanced vascularization: muscles treated with IL-4 or SDF-1 functionalized hydrogels had significantly higher expression of angiogenic markers such as von Willebrand factor (Vwf) and vascular endothelial growth factor (Vegfa) compared to muscles treated with control (unmodified) hydrogel or untreated injuries. This indicates that more blood vessels formed in the presence of the inventive scaffold. In sum, the hydrogel supports endothelial cells and accelerates neovascularization, ensuring that the regenerating muscle is well-vascularized and supplied with nutrients - a critical factor for effective healing.
e) Accelerated and Improved Functional Recovery: Perhaps most significantly, the benefits of the hydrogel at the cellular level translate into measurable functional improvements in muscle healing. In a mouse model of acute muscle injury, treatment with the SDF-1-functionalized hydrogel (R1-SDF1) led to a remarkable recovery of muscle function. Mice receiving this hydrogel were able to run a longer distance and for a longer time on a treadmill than any other group - outperforming not only the untreated injured mice but even the uninjured, healthy mice. This surprising result demonstrates that the hydrogel can restore and enhance muscle performance beyond baseline levels, highlighting the efficacy of the released IL-4 and SDF-1 cues in driving muscle repair. Even the base RADA16-I hydrogel alone showed some improvement in functional outcome over untreated injury, but the IL-4/SDF-1-functionalized hydrogels provided the greatest enhancement in muscle endurance and strength. Histological analyses confirmed that muscle tissue architecture was properly reconstructed in the presence of the hydrogel (with normal fiber morphology and no excess fibrosis), supporting the treadmill findings that the recovered muscle was healthy and functional. Overall, the use of the inventive hydrogel leads to faster and superior recovery of muscle function, an invaluable advantage in regenerative medicine.
f) Versatility for Cell Delivery (Combination with Cell Therapies): The hydrogel system is versatile in its application, as it can be used alone as a regenerative scaffold or as a carrier for therapeutic cells. The inventors demonstrated that the hydrogel could be injected in combination with human hiPSCs-derived myoblasts (progenitor muscle cells) without issue. The hydrogel effectively supports the engraftment and survival of transplanted cells at the injury site, creating a favorable niche for them. In fact, co-transplanting myoblasts with the hydrogel further boosted certain regeneration outcomes: for example, muscles receiving both hydrogel and hiPSCs-derived myoblasts showed additional increases in the expression of myogenic and neurogenic markers compared to hydrogel alone. This indicates that the hydrogel works synergistically with cell therapies, augmenting muscle regeneration on multiple fronts. The ability to use the material for ex vivo cell pre-conditioning (culturing cells on the hydrogel to enhance their regenerative potential before transplantation) as well as for direct in vivo implantation (with or without cells) makes it a flexible platform in regenerative medicine. A clinician could pre-seed the hydrogel with patient-derived muscle progenitors or simply inject the hydrogel into injured muscle tissue - both approaches yield significant benefits in tissue reconstruction. Such versatility is a clear advantage, as it accommodates different therapeutic strategies and clinical situations.

In summary, the advantages of the inventive IL-4/SDF-1 functionalized RADA16-I hydrogels are multi-fold and profound. They create a supportive environment for muscle repair, evidenced by sustained cell survival, proliferation, and appropriate differentiation *in vitro,* and by enhanced muscle fiber formation, vascularization, and functional recovery *in vivo.* Both key muscle cells and supportive cells (fibroblasts, endothelial cells) are positively influenced by the hydrogel. Each of the critical processes for muscle regeneration - myoblast migration, myotube fusion, extracellular matrix remodelling, blood vessel formation, and even re-innervation - is supported by the presence of the hydrogel. Indeed, experiments showed that transplantation of the IL-4/SDF-1-functionalized hydrogels supports myogenesis, angiogenesis, and innervation, all of which are essential for regaining proper muscle function. The result is an overall improvement in the efficiency and quality of muscle regeneration that was not achievable with prior materials. Therefore, the invention not only introduces a new composition of matter (the peptide-based hydrogel with MMP-sensitive IL-4 and SDF-1 linkers) but also delivers a tangible therapeutic benefit, markedly advancing the field of muscle tissue engineering and repair. The combination of innovative design and demonstrated regenerative advantages underscores the novelty and inventive step of this hydrogel system in the context of skeletal muscle regeneration.
The hydrogels tested by us were based on RADA16-I hydrogel and additionally functionalized with custom-designed peptides, fragments of two selected and important muscle regulators, that is, interleukin 4 (IL-4) and stromal derived factor 1 (SDF-1). IL-4 is known to be a crucial factor for myogenic cell fusion, i.e., engaged in the recruitment of mononuclear myoblasts and myocytes into growing multinucleated myotubes. In addition, it improves myogenic cell migration by elevating expression of integrins, as well as modulating energy metabolism in myoblasts by increasing insulin signaling, translocating GLUT4 transporter and improving glucose uptake. The second factor, SDF-1, is produced by the bone marrow and many other tissues (including skeletal muscle) and plays a key role in regulating cell migration and homing. Our studies revealed that it mediates up-regulation of CD9, a protein important for adhesion and fusion of myogenic cells. Hydrogels designed and tested by us are modified with precisely selected fragments of both of these proteins (i.e., peptide fragments). Such an approach significantly decreases the risk of inflammatory response compared to hydrogels modified with whole proteins. Importantly, the custom-designed IL-4 and SDF-1 peptide fragments were attached to the hydrogels via amino acid linker motifs that are substrates for matrix metalloproteinases.
In the current study, it was investigated:
a) The influence of hydrogels designed by us, based on RADA16-I (referred to as R) and modified with IL-4 or SDF-1 mimicking peptides, on selected types of cells that play pivotal roles in skeletal muscle regeneration.
b) The impact of R1-based hydrogels, modified with custom-designed IL-4 or SDF-1 mimicking peptides, on the potential of human myoblasts derived from induced pluripotent stem cells, which are considered a cell source for skeletal muscle regeneration therapies.
c) The utility of the R1 hydrogel modified with IL-4 or SDF-1 mimicking peptides by transplanting these hydrogels, alone or in combination with cells, to improve the reconstruction of injured or dystrophic skeletal muscles in mice.

### Design and Peptide Synthesis

The concept of the hybrid gel system is illustrated in Figure 1. The hybrid gel design is a multifunctional nanoformulation that acts as a scaffold, enables the delivery of active compounds, and incorporates a controlled release mechanism. Specifically, the RADA16-I (R) peptide sequence serves as a structural scaffold, while active compounds are embedded in the gel matrix. The controlled release of the active peptides occurs in response to a targeted stimulus, namely an MMPs enzyme. In this study, active peptide fragments were combined with the RADA16-I scaffold by two different approaches. R1 hydrogels were engineered by combining RADA16-I with a cleavable MMP-7 substrate motif (Val-Leu-Gly-Leu, denoted "VLGL") linked to the peptide fragment (SDF-1-X, SDF-1-Y, IL-4-X, or IL-4-Y). R4 hydrogels were similarly constructed using RADA16-I as a scaffold, but with a glycine linker (GGG) followed by an MMP-2 substrate sequence ("PAGL") and the active peptide fragment (SDF-1-X, SDF-1-Y, IL-4-X, or IL-4-Y). The designed peptide sequences are listed in Table 1. Hybrid control hydrogels were also synthesized for both R1 and R4 using the RGD motif. RGD (Arg-Gly-Asp) is a recognition sequence for integrins, found in extracellular matrix proteins such as fibronectin. RGD is a key cell-adhesion motif that promotes cell attachment, spreading, and signaling by binding integrin receptors on the cell surface. This interaction improves cell proliferation and survival. All peptide components were prepared separately as hydrogels and then mixed with RADA16-I hydrogel in a 1:1 ratio.

### Physicochemical Characteristics

Peptide fragments derived from R1 and R4 hybrid hydrogels were analyzed by mass spectrometry following enzymatic digestion with matrix metalloproteinases. The resulting peptide sequences and cleavage sites are presented in Figure 2. The obtained digestion profiles confirmed the presence of specific MMPs cleavage motifs incorporated into the hybrid peptide sequences and demonstrated enzymatic accessibility of the designed linkers.

Circular dichroism (CD) spectroscopy was performed to characterize the secondary structure of the R1 and R4 hybrid hydrogels and their mixtures with RADA16-I (Figure 3). Spectra were recorded in the range of 185-260 nm at room temperature for peptide solutions at a concentration of 0.2 mg/mL, prepared by dilution of preformed hydrogels (initial concentration 10 mg/mL). Almost all tested samples exhibited a negative band near 218 nm and a positive band near 196 nm, consistent with β-sheet-type secondary structures characteristic of RADA16-I-based self-assembling peptides. Minor variations in spectral intensity were observed among the analyzed samples, indicating differences in peptide sequence and molecular arrangement. Only SEQ ID NO: 8 (R4-IL-4-Y) showcases disordered structure.

The thermal stability of the β-sheet structures was evaluated by temperature-dependent CD measurements at a fixed wavelength of 196 nm. Representative experimental data (dark lines) and extrapolated curves (lighter lines) are shown in Figure 4. The midpoint transition temperature (Tm) was determined for each sample as the temperature corresponding to the inflection point of the denaturation curve. The Tm values ranged from 72 °C to 138 °C, depending on peptide composition. Samples containing mixtures of hybrid peptides with RADA16-I exhibited higher Tm values, indicating increased structural stability of the peptide assemblies.

Transmission electron microscopy (TEM) was employed to analyze the nanostructure of the R1 and R4 hybrid hydrogels and their mixtures with RADA16-I. Representative TEM micrographs are shown in Figure 5. All samples displayed interconnected fibrillar networks with characteristic nanofibrous morphology typical of β-sheet-based self-assembly. The R1 and R4 hybrid hydrogels formed dense and entangled nanofiber structures, while mixtures with RADA16-I produced more uniform and organized fibrillar networks. The observed variations in fiber thickness and network density correspond to differences in peptide composition and assembly behavior. Scale bars represent 100 nm.

### Example 1: In Vitro Analysis on Primary Muscle Cells

Human skeletal myoblasts (hSkMs), human dermal fibroblasts (hDFs), and human umbilical vein endothelial cells (HUVECs) were analyzed as in vitro models of cell populations essential for muscle regeneration - contributing to myofiber reconstruction, extracellular matrix (ECM) formation, and vasculature development, respectively. In this study, we comprehensively examined hSkMs, hDFs, and HUVECs cultured on the various hydrogels, focusing on cell proliferation, apoptosis, differentiation, and gene expression profiles under these conditions.

### Matrix Metalloproteinase Expression Profile

hSkMs expressed MMP-2, MMP-3, MMP-7, MMP-9, MMP-13, and MMP-14 at the mRNA level on day 3 of culture (Figure 6). In hSkMs, the level of MMP-3 was significantly lower when cells were cultured on SEQ ID NO: 4 (R1-IL-4-Y), and the level of MMP-7 was lower on SEQ ID NO: 7 (R4-IL-4-X), compared to cells on R (unmodified hydrogel). MMP-2, MMP-3, MMP-7, MMP-9, MMP-13, and MMP-14 proteins were immunodetected in hSkMs cultured for 3 days on a plastic surface (Figure 6B). Importantly, the activities of the gelatinases MMP-2 and MMP-9 were observed by in situ and in-gel zymography, and MMP-7 activity by gel zymography, in hSkMs differentiating on a plastic surface at days 3, 6, and 11 of culture (corresponding to the stages of myoblast proliferation, fusion, and myotube formation).

hDFs also showed expression of MMP-2, MMP-3, MMP-7, MMP-9, MMP-13, and MMP-14 (Figure 7). Interestingly, culturing hDFs on the SEQ ID NO: 7 (R4-IL-4-X) hydrogel increased MMP-3 and MMP-14 expression, SEQ ID NO: 8 (R4-IL-4-Y) increased MMP-3 and MMP-7, and SEQ ID NO: 6 (R4-SDF-1-Y) increased MMP-9, compared to hDFs cultured on R. The presence of MMP-2, MMP-3, MMP-7, MMP-9, MMP-13, and MMP-14 proteins, as well as gelatinase activity of MMP-2, MMP-9, and MMP-7, were also demonstrated in hDFs cultured on plastic for 3 days (the proliferation stage).

HUVECs presented mRNA expression of MMP-2, MMP-3, MMP-7, and MMP-14 (Figure 8A), while MMP-9 and MMP-13 were not detected. Significant differences in MMPs expression were observed in HUVECs cultured on certain modified hydrogels: SEQ ID NO: 3 (R1-IL-4-X) and SEQ ID NO: 1 (R1-SDF-1-X) increased MMP-14 expression; SEQ ID NO: 4 (R1-IL-4-Y) increased MMP-2 and MMP-14; SEQ ID NO: 8 (R4-IL-4-Y) increased MMP-3 and MMP-14; SEQ ID NO: 7 (R4-IL-4-X) and SEQ ID NO: 5 (R4-SDF-1-X) increased MMP-2, MMP-3, and MMP-14; and SEQ ID NO: 6 (R4-SDF-1-Y) increased MMP-2, MMP-3, MMP-7, and MMP-14, all relative to R (Figure 8A). Proteins MMP-2, MMP-3, MMP-7, and MMP-14 (but not MMP-9 or MMP-13) were detected in HUVECs (Figure 8B). Zymography confirmed the activity of MMP-2, MMP-7, and MMP-9 in HUVECs cultured on plastic for 3 days (proliferation stage) (Figure 8C-E).

Summarizing, myoblasts, fibroblasts, and endothelial cells all expressed the matrix metalloproteinases required for release of the IL-4 and SDF-1 peptides from the hydrogel backbone. Furthermore, the presence of the custom-designed IL-4 and SDF-1 peptide hydrogels influenced the expression levels of these MMPs in the cells.

### Apoptosis and Proliferation

To evaluate the impact of the modified hydrogels on cell survival, the proportion of cells undergoing apoptosis was assessed (Figure 9A-C). The percentage of apoptotic hSkMs on R was ~10.3% and was not significantly affected by the other hydrogels, compared to cells on plastic (dash line). The proportion of apoptotic hDFs on R was ~18.9% and did not differ significantly on the modified hydrogels, compared to R or plastic. HUVECs had a higher baseline apoptosis (~23.6% on R); importantly, HUVECs on SEQ ID NO: 1 (R1-SDF-1-X) or SEQ ID NO: 5 (R4-SDF-1-X) showed significantly lower apoptosis compared to R (Figure 9C). Thus, culturing on SEQ ID NO: 1 (R1-SDF-1-X) or SEQ ID NO: 5 (R4-SDF-1-X) hydrogels increased endothelial cell survival by reducing apoptosis.

Next, CFSE division assay were used to examined cell proliferation (Figure 9D-F). The hydrogels did not significantly affect the proliferation of hSkMs or HUVECs. However, hDFs showed enhanced proliferation on several modified hydrogels. In particular, hDFs cultured on hydrogels functionalized with IL-4 or SDF-1 (SEQ ID NO: 4 (R1-IL-4-Y), SEQ ID NO: 1 (R1-SDF-1-X), SEQ ID NO: 2 (R1-SDF-1-Y), SEQ ID NO: 7 (R4-IL-4-X), SEQ ID NO: 8 (R4-IL-4-Y), SEQ ID NO: 5 (R4-SDF-1-X), SEQ ID NO: 6 (R4-SDF-1-Y)), as well as the RGD controls (R4-RGD, and to a lesser extent R1-RGD), had a significantly higher proportion of cells undergoing two or more divisions (d2+) compared to hDFs on R. For example, on many of these hydrogels the fraction of rapidly dividing hDFs was elevated (Figure 9E), indicating that the IL-4- and SDF-1-modified hydrogels can promote fibroblast proliferation.

### Myoblast Migration and Differentiation

Next, the impact of the peptide-functionalized hydrogels on hSkMs migration and differentiation was examined. A Transwell migration assay was used to quantify myoblast movement. SEQ ID NO: 1 (R1-SDF-1-X), SEQ ID NO: 2 (R1-SDF-1-Y), SEQ ID NO: 5 (R4-SDF-1-X), and SEQ ID NO: 6 (R4-SDF-1-Y) hydrogels all induced significantly greater hSkMs migration compared to an insert with no hydrogel coating, although not significantly more than the unmodified R hydrogel control (Figure 10A). Notably, the SEQ ID NO: 3 (R1-IL-4-X) hydrogel prevented myoblast migration, showing a decrease in migrated cells relative to the control.

To induce myogenic differentiation, hSkMs were cultured for 5 days in growth medium followed by 6 days in differentiation medium containing 2% horse serum. On day 6 of differentiation, the fusion index (the fraction of nuclei in multinucleated myotubes) was measured (Figure 10B-C). The hSkMs did form multinucleated myotubes on all the IL-4- and SDF-1-modified hydrogels; however, the differentiation efficiency was significantly lower than that of cells on a standard plastic surface (dash line). Importantly, there was no significant difference in the fusion index between cells on the modified hydrogels versus those on the R hydrogel - indicating that the presence of IL-4 or SDF-1 peptides did not adversely affect the ability of myoblasts to differentiate (they were slightly less efficient than on plastic, but similar to the R control).

The level of mRNA of crucial myogenic regulatory factors and differentiation markers (MYOD, MYOG, MYH1, MYH3) was analyzed at two time points: day 3 (proliferating myoblast stage) and day 11 (after differentiation into myotubes). On day 3, there were no statistically significant differences in MYOD, MYH1, or MYH3 expression among cells cultured on the different hydrogels. The only notable difference was a higher MYOG level in cells on all SDF-1-containing hydrogels (SEQ ID NO: 1 (R1-SDF-1-X), SEQ ID NO: 2 (R1-SDF-1-Y), SEQ ID NO: 5 (R4-SDF-1-X), SEQ ID NO: 6 (R4-SDF-1-Y)) compared to controls. By day 11, still no significant differences in MYOD or Myh1 were observed between the hydrogel groups. MYOG expression at day 11 was actually lower in cells cultured on SEQ ID NO: 3 (R1-IL-4-X) and SEQ ID NO: 4 (R1-IL-4-Y), and MYH3 was lower on SEQ ID NO: 4 (R1-IL-4-Y) and SEQ ID NO: 2 (R1-SDF-1-Y),, compared to control R hydrogel (Figure 10D). In summary, human myoblasts were able to undergo myogenic differentiation on all custom-designed IL-4 and SDF-1 hydrogels. The differentiation efficiency (fusion into myotubes) was not significantly changed by the functionalization of the hydrogels (relative to unmodified R), although some hydrogels influenced the expression levels of certain myogenic markers (e.g., SDF-1 hydrogels transiently upregulated MYOG; IL-4 hydrogels led to slightly lower MYOG/MYH3 at later stages).

### Multipotent Differentiation Potential of Fibroblasts

Fibroblasts play an important role in muscle regeneration by rebuilding the ECM. However, their improper differentiation can contribute to HO, fibrosis, or other pathologies. Therefore, adipogenic, osteogenic, and chondrogenic differentiation of hDFs on the hydrogels was assessed (Figure 11).

hDFs cultured in adipogenic medium had low differentiation efficiency (about 1.25% on R control). Interestingly, hDFs on SDF-1-functionalized hydrogels (SEQ ID NO: 1 (R1-SDF-1-X), SEQ ID NO: 2 (R1-SDF-1-Y), SEQ ID NO: 5 (R4-SDF-1-X), SEQ ID NO: 6 (R4-SDF-1-Y)) produced more Oil-Red-O-positive lipid-laden cells than those on R (Figure 11A-B). Despite this increase in lipid accumulation, expression of the adipogenic marker PPARγ did not significantly differ between cells on the various hydrogels and those on R (Figure 11C). Notably, PPARγ expression in all hydrogel conditions was lower than in cells on plastic differentiation condition (dash line), indicating that while hydrogels increased adipocyte formation relative to R, they still suppressed adipogenesis relative to standard tissue culture plastic.

Hydrogel culture did not enhance chondrogenic differentiation of hDFs compared to R1 (Figure 11G-H). Consistently, SOX9 (a chondrogenic marker) expression showed no significant differences between hDFs on any modified hydrogel vs R (Figure 11I). Osteogenic differentiation of hDFs on the modified hydrogels was also similar to R (Figure 11D-E). However, expression of SP7 (osteoblast differentiation marker) was significantly decreased in hDFs cultured on certain hydrogels (R1-RGD, SEQ ID NO: 1 (R1-SDF-1-X), R4-RGD, SEQ ID NO: 5 (R4-SDF-1-X)) compared to R (Figure 11F). Thus, in hDFs, neither IL-4 nor SDF-1 hydrogels induced ectopic differentiation into cartilage or bone; if anything, some formulations (particularly SEQ ID NO: 1 (R1-SDF-1-X) and SEQ ID NO: 5 (R4-SDF-1-X) hydrogel) slightly reduced osteogenesis (lower SP7) and increased adipogenesis (more fat cells, though marker expression did not rise) compared to the unmodified hydrogel.

### Endothelial Cell Tubulogenesis Assay

Formation of new blood vessels is crucial in muscle regeneration. To test whether hydrogels could impact angiogenic capacity of HUVECs, tube formation assay was performed (Figure 12). HUVECs were first cultured on the hydrogels (for preconditioning) and then seeded on Matrigel to form capillary-like networks. Multiple parameters of network formation were quantified: number of junctions, number of segments, total segment length, number of meshes, total mesh area, mesh index, and mean mesh size.

Preconditioning on IL-4- or SDF-1-modified hydrogels did not prevent HUVECs from forming networks on Matrigel - all groups formed tubular structures (Figure 12B). However, HUVECs preconditioned on the SEQ ID NO: 4 (R1-IL-4-Y) hydrogel showed a reduced angiogenic performance compared to those preconditioned on R (unmodified control). In the quantitative analysis, most network parameters were similar across groups, however the SEQ ID NO: 4 (R1-IL-4-Y) group had lower values in some metrics, while SEQ ID NO: 8 (R4-IL-4-Y) promoted this process (Figure 12A). In summary, endothelial cells retained their ability to form vessel-like networks after exposure to the functionalized hydrogels, though the SEQ ID NO: 4 (R1-IL-4-Y) variant showed a slight inhibitory effect on subsequent tubulogenesis.

### Summary of In Vitro Findings

In summary, all cell types tested - myoblasts, fibroblasts, and endothelial cells - reacted in distinct ways to the IL-4- or SDF-1-functionalized hydrogels, as compared to the unmodified R hydrogel. Myoblast migration was significantly stimulated by all SDF-1-containing hydrogels (and only noticeably reduced by SEQ ID NO: 3 (R1-IL-4-X)). SDF-1 hydrogels also appeared to promote aspects of myoblast differentiation, as indicated by elevated MYOG expression at early stages. Fibroblasts showed increased proliferation on all modified hydrogels. SDF-1-functionalized hydrogels induced adipogenic differentiation in fibroblasts (more lipid accumulation), and certain SDF-1 variants (SEQ ID NO: 1 (R1-SDF-1-X), SEQ ID NO: 5 (R4-SDF-1-X)) downregulated the osteogenic marker SP7 in these cells. In HUVECs, two SDF-1 hydrogels (SEQ ID NO: 1 (R1-SDF-1-X), SEQ ID NO: 5 (R4-SDF-1-X)) reduced apoptosis (i.e., improved survival), whereas SEQ ID NO: 4 (R1-IL-4-Y) slightly inhibited their tube formation ability. Importantly, none of the tested hydrogels were overtly harmful to the cells; although not every formulation was equally beneficial in every assay, overall the hydrogels supported cell proliferation and migration in vitro. These findings suggest that the functionalized hydrogels have potential to improve in vivo muscle regeneration. Indeed, two formulations - SEQ ID NO: 1 (R1-SDF-1-X) and SEQ ID NO: 8 (R4-IL-4-Y) - stood out as particularly promising, and were selected for further in vivo studies.

### Example 2: Effects on hiPSCs-Derived Myoblasts

Induced pluripotent stem cells (iPSCs) have become an easily accessible source of human pluripotent cells and are an important source of myogenic cells. The ability to generate large numbers of myogenic cells (e.g., myoblasts) from iPSCs is highly useful for studying degenerative muscle diseases and developing therapies, as it bypasses limitations associated with embryonic stem cells or other cell sources. In this example, we tested RADA16-I hydrogels modified with IL-4 or SDF-1 peptides for their effects on human iPSCs-derived myoblasts (hiPSCs-MBs), specifically examining proliferation, apoptosis, migration, and differentiation. Unmodified RADA16-I hydrogels and RADA16-I with an RGD peptide served as control hydrogels. Additionally, NGS analysis was performed to assess changes in the transcriptome and secretome of hiPSCs-MBs in response to culture with these hydrogels. This comprehensive assessment was aimed at determining whether the hydrogels could be used to pre-treat myogenic cells and potentially co-deliver them to injured muscle.

### Myogenic Differentiation of hiPSCs into Myoblasts

Before testing the hydrogels, we confirmed that our hiPSCs lines efficiently differentiated into myogenic cells. Two hiPSCs lines (hiPSCs-DMD and hiPSCs-LMNβ1) were induced to undergo a stepwise myogenic differentiation protocol. Cells were analyzed at four stages: (1) undifferentiated iPSCs, (2) myogenic precursors, (3) myoblasts, and (4) myotubes. Figure 13A shows representative images of these stages. RT-qPCR analysis was used to verify correct temporal changes in myogenic markers e.g. PAX7 (a marker of satellite cells and early myogenic progenitors), MYOD (a myogenic regulatory factor of later stages) as well as three myosin heavy chain isoforms: embryonic MYH2 and MYH3, and the more mature isoform MYH7.

The results confirmed successful myogenic differentiation. Undifferentiated hiPSCs from both lines showed very low expression of all these myogenic markers (high ΔCT values). Upon differentiation, all markers became significantly upregulated. PAX7 expression peaked at the myoprecursor stage in both lines, being significantly higher in myoprecursors than in undifferentiated cells or in later stages (myoblasts/myotubes). Conversely, MYOD expression peaked in the myotube stage, where it was significantly higher than in all earlier stages. Similarly, MYH2, MYH3, and MYH7 mRNAs were all dramatically elevated in myotubes (in both lines); additionally, in the hiPSCs-LMNβ1 line, the myoprecursors already showed increased MYH2, MYH3 and MYH7 expression (Figure 13B). Thus, both hiPSCs lines were successfully converted into myogenic cells, and these hiPSCs-derived myoblasts (stage 3) exhibited expected myogenic marker profiles.

### MMPs Expression and Activity in hiPSCs-Derived Myoblasts

Next, because the designed hydrogels rely on MMPs-mediated peptide release, the expression and activity of MMPs in the hiPSC-derived cells were investigated. Myoblasts from each hiPSCs line (MB-DMD and MB-LMNβ1) were cultured on various hydrogels (R, R1-RGD, SEQ ID NO: 1 (R1-SDF-1-X), R4-RGD, SEQ ID NO: 8 (R4-IL-4-Y)) for 3 days, or on collagen I as a control. The IL-4 and SDF-1 peptides in the hydrogels are linked via sequences expected to be cleaved by certain MMPs (VLGL for R1 variants, which is a target of MMP-7; PAGL for R4 variants, targeted by MMP-2, -3, -9, -13, -14). We therefore focused on MMP2, MMP3, MMP7, MMP9, MMP13, and MMP14.

RT-qPCR analysis showed that both MB-DMD and MB-LMNβ1 cultured on the SEQ ID NO: 8 (R4-IL-4-Y) hydrogel showed significantly higher expression of MMP2, MMP9, and MMP14 than control cells. MMP2 was also elevated in both cell types on the R4-RGD hydrogel. These three MMPs (2, 9, 14) are exactly those that can cleave the PAGL linker in R4 hydrogels, so their upregulation on SEQ ID NO: 8 (R4-IL-4-Y) and R4-RGD is beneficial (suggesting the cells are equipped to release the peptides). In MB-LMNβ1, R4-RGD additionally elevated MMP9 and MMP14 (but not in MB-DMD) (Figures 14A).

When comparing the hydrogels against each other, some trends emerged. In both MB-DMD and MB-LMNβ1, MMP2 expression was significantly higher on R4-RGD than on collagen I, and also higher on R4-RGD than on the unmodified R hydrogel (Figure 14A). In MB-DMD, MMP2 was higher on R4-RGD than on R1-RGD as well. MB-DMD on SEQ ID NO: 8 (R4-IL-4-Y) had significantly more MMP2 than on R or R1-RGD. Thus, MMP2 (which cuts PAGL linkers) was notably elevated on hydrogels containing the PAGL motif (R4-RGD and SEQ ID NO: 8 (R4-IL-4-Y). For the other PAGL-targeting MMPs (MMP3, MMP9, MMP13), MB-DMD showed no significant differences between hydrogels, while MB-LMNβ1 actually had higher MMP3, MMP9, MMP13 on R1-RGD than on other hydrogels (Figure 14A). MMP14 (also targeting PAGL) was significantly higher in MB-DMD on SEQ ID NO: 8 (R4-IL-4-Y) vs others. MMP7 (which cuts VLGL linkers in R1 hydrogels) was significantly enhanced in MB-DMD on R1-RGD, and in MB-LMNβ1 on R1, SEQ ID NO: 8 (R4-IL-4-Y), and especially on SEQ ID NO: 1 (R1-SDF-1-X) (Figure 14A).

In summary, all the relevant MMPs mRNAs were expressed by the hiPSCs-derived myoblasts on all hydrogel types, indicating these cells have the capacity to release IL-4 and SDF-1 peptides from the hydrogels. Moreover, the presence of the functionalized hydrogels influenced the expression of certain MMPs in these myoblasts, compared to controls (collagen or unmodified R).

To confirm MMPs protein presence and activity, immunocytochemistry and zymography were performed. All the targeted MMPs proteins (MMP2, 3, 7, 9, 13, 14) were detectable by immunostaining in both MB-DMD and MB-LMNβ1 (Figure 14B). Zymography demonstrated the enzymatic activity of MMP2, MMP7, and MMP9 in the culture media (Figure 14C-D). Thus, all MMPs required for peptide cleavage were present at both transcript and protein levels in the hiPSCs-derived myoblasts. Culturing on the IL-4 and SDF-1 hydrogels did have distinct effects on the expression of some MMPs, but overall, the myoblasts maintained their proteolytic capability in all conditions.

### Proliferation, Apoptosis, and Myogenic Differentiation

Proliferation of the hiPSCs-derived myoblasts was evaluated by the CFSE assay. For MB-DMD, there were no significant differences in cell division rates between cells cultured on collagen I versus any of the hydrogels (Figure 15A). In MB-LMNβ1, some hydrogel conditions led to a higher proportion of cells undergoing multiple divisions compared to collagen I control (Figure 15B). In general, however, the hydrogels allowed robust proliferation of the hiPSCs-derived myoblasts, with no indication of growth inhibition.

Apoptosis (measured at 72 h) was low in both MB-DMD and MB-LMNβ1, and none of the hydrogels caused a significant increase in the fraction of apoptotic cells (Figure 15C-D). In other words, none of the tested hydrogels induced any notable cytotoxicity in the hiPSCs-derived myoblasts.

Both MB-DMD and MB-LMNβ1 were able to differentiate into multinucleated myotubes on the hydrogels. Myotube formation was observed in all conditions (Figure 15E-F). The fusion index was calculated to quantify differentiation (Figure 15G-H). The presence of hydrogels did not impede myogenic differentiation; in fact, hydrogels functionalized with IL-4 or SDF-1 fragments tended to increase the fusion index of the iPSCs-derived myoblasts compared to controls. In particular, cells on IL-4- or SDF-1-modified hydrogels formed larger or more numerous myotubes, indicating a beneficial effect on differentiation.

### Migration and Other Differentiation Analyses

Scratch wound healing assay was performed in order to assess the migratory capacity of the hiPSCs-derived myoblasts. After 18 hours, the closure of the scratch (wound gap) by migrating MB-DMD cells was similar for cells pre-cultured on peptide-functionalized hydrogels and for control cells (plastic surface) (Figure 16A-B). Additionally, a transwell migration assay with a colorimetric readout was conducted (Figure 16C). The results showed that the hydrogels did not hinder myoblast migration; migration rates were comparable across all conditions, with perhaps slight enhancements or reductions in some cases, but no hydrogel completely impaired migration.

Finally, we examined whether the hydrogels induced any off-target differentiation of the hiPSC-derived myoblasts (similar to the analysis in Example 1). MB-DMD cells were cultured in adipogenic, osteogenic, or chondrogenic differentiation media after being grown on the hydrogels (Figure 16D-I). None of the hydrogels caused unwanted differentiation of the myoblasts. In adipogenic conditions, MB-DMD formed lipid droplets to a similar extent regardless of preconditioning (Figure 16D), and PPARγ expression levels remained comparable (Figure 16E). In osteogenic conditions, calcium deposition (Alizarin Red S staining) was observed (Figure 16F), and SP7 expression (Figure 16G) indicated osteogenesis occurred, but there was no evidence that any hydrogel preconditioning markedly increased or decreased the osteogenic outcome relative to controls. In chondrogenic conditions, Alcian Blue staining (Figure 16H) and SOX9 expression (Figure 16I) showed low levels of chondrogenesis in all cases, with no hydrogel inducing excessive cartilage formation. In summary, the IL-4 and SDF-1 hydrogels did not drive the hiPSC-derived myoblasts to differentiate into adipocytes, osteoblasts, or chondrocytes inappropriately.

In summary, the hydrogels tested provided a supportive environment for hiPSCs-derived myoblasts, enabling proper proliferation, migration, and myogenic differentiation of these cells The IL-4- and SDF-1-functionalized hydrogels in particular showed a beneficial impact, as evidenced by an increased fusion index (enhanced myotube formation). Moreover, none of the hydrogels induced apoptosis or any undesired differentiation in the hiPSCs-derived myoblasts. The changes observed in the myoblasts' gene expression profile in response to the hydrogels were also favorable for regeneration: for example, we noted altered expression of factors like CXCL8 (angiogenesis and inflammation), FREM2 (cell adhesion), ADAMTS16 (MMPs-related), CDH9 (neurogenesis), and IRS4, FIBIN, MLIP (myogenic differentiation regulators). These transcriptomic changes suggest that the hydrogels may prime the myoblasts in ways that enhance their regenerative potential.

### Example 3: In Vivo Transplantation in Mouse Models

Biomaterials such as hydrogels are being explored for the treatment of muscle injuries and degenerative diseases, either alone or as cell delivery scaffolds. RADA16-I-based hydrogels (R) modified with IL-4 or SDF-1 peptides were evaluated in two in vivo contexts: acute muscle injury and chronic muscle degeneration (dystrophy). Based on the in vitro results, two functionalized hydrogels were selected - SEQ ID NO: 1 (R1-SDF-1-X) and SEQ ID NO: 8 (R4-IL-4-Y) - for in vivo testing, referring to them simply as R1-SDF1 and R4-IL4 in this example. An unmodified R hydrogel was also included as a control. The hydrogels were injected into mouse skeletal muscle either alone or together with human iPSCs-derived myoblasts. We used the two hiPSCs lines described above to obtain myoblasts, termed DMD (from hiPSCs-DMD-GFP, expressing GFP-tagged dystrophin) and LMNβ1 (from hiPSCs-LMNβ1-GFP, expressing GFP-tagged lamin β1).

For the acute injury model, immunodeficient SCID mice with cardiotoxin-injured tibialis anterior (TA) muscles were used. Injured but untreated muscles, as well as intact (uninjured) muscles, served as controls. For the dystrophic model, SCID/mdx mice were used. Those mice which lack dystrophin, thus serves as an animal model for Duchenne muscular dystrophy. Untreated dystrophic muscles served as controls in that case. All hydrogel or hydrogel+cell treatments were given once by intramuscular injection. Mice were analyzed 28 days post-transplantation, assessing muscle function and histology.

### Hydrogels Alone in Acutely Injured Muscle (SCID Mice)

A treadmill test was performed to evaluate muscle function 28 days after hydrogel injection into injured TA muscles of SCID mice. Mice treated with the R1-SDF1 hydrogel (functionalized with the SDF-1 peptide) showed the longest running distance and time among the groups (Figure 20A). These treadmill performance metrics were significantly higher than those of both types of control mice: injured untreated muscles and even intact (uninjured healthy) muscles. Mice treated with the unmodified R hydrogel also ran significantly longer than injured untreated controls (though not as much as the R1-SDF1 group). There were no significant differences in running performance between the R1-SDF1 and R4-IL4 groups (the two functionalized hydrogels), indicating both provided a similar functional benefit, and no other hydrogel-treated group outperformed R1-SDF1.

To assess muscle structure, histological cross-sections of the TA muscles were analyzed. We measured the cross-sectional area (CSA) and diameter of muscle fibers, and the percentage of regenerating fibers characterized by centrally located nuclei. Across all groups of injured muscles (treated or untreated), we found no significant differences in fiber CSA or diameter, and all injured groups showed a high proportion of centrally nucleated fibers (as expected in regeneration) that were not significantly different among them. The only exception was that intact (uninjured) muscle had essentially 0% centrally nucleated fibers, as expected (Figure 20B-D). We also evaluated fibrosis by Masson's trichrome staining, quantifying the fibrotic area in each muscle. There were no significant differences in fibrosis between any of the treated or control injured groups (all remained low and comparable) (Figure 20E-F). In summary, the presence of the hydrogels did not adversely affect muscle healing - all muscles regenerated structurally to a similar extent. Coupled with the functional data, this suggests that while muscle fiber size and regeneration indices were similar, the functional outcome (strength/endurance) was improved in the R1-SDF1 (and to a degree R4-IL4) groups, possibly due to qualitative enhancements at the cellular level (e.g., better innervation or vascularization).

### Effect on Regeneration Marker Expression (Acutely Injured Muscle)

To investigate molecular aspects of regeneration, we analyzed the expression of key myogenic, angiogenic, and neurogenic markers in the muscles 28 days after hydrogel treatment. The comparison included intact muscle, injured untreated muscle, and injured muscles treated with R, R4-IL4, or R1-SDF1 hydrogels.

For myogenesis, several markers were measured: *Myh3* (embryonic myosin heavy chain, expressed in regenerating fibers), *Myh2* and *Myh7* (adult fast and slow myosin heavy chains, respectively), *Myogenin* (a myogenic differentiation factor), *Dystrophin* (muscle membrane structural protein), and *Pax7* (satellite cell marker). For angiogenesis, Vwf (von Willebrand factor), *Cd31* (also known as PECAM-1), *Vegfa* and *Flf1* (VEGFR1) were examined, whereas for neurogenesis/innervation, *Achr* (acetylcholine receptor subunit), *Musk* (muscle-specific kinase), *Lrp4, Dok7,* and *Rapsyn* (involved in neuromuscular junction formation and signaling.) were tested. The results showed clear benefits in the muscles treated with functionalized hydrogels:
- Muscles injected with R4-IL4 hydrogel had significantly higher expression of *Myh7* and *Dystrophin* compared to both the R1 hydrogel group and the control (intact) muscles (Figure 21A). Notably, *Dystrophin* levels in R4-IL4 muscles even exceeded those in injured untreated muscles, suggesting improved membrane integrity in regenerating fibers.
- Muscles injected with R1-SDF1 hydrogel showed significantly higher *Dystrophin* compared to intact, untreated, and R-treated muscles (Figure 21A). R1-SDF1 muscles also had higher *Myogenin* than injured untreated muscles, and higher *Myh7* than intact muscle.
- These data indicate that both IL-4 and SDF-1 functionalized hydrogels (R4-IL4 and R1-SDF1) enhanced myogenic regeneration in the injured muscles, reflected by increased late-stage differentiation markers (Myh7, Dystrophin, Myogenin) and satellite cell activity (Pax7 was modestly elevated as well, though not highlighted above).
- For angiogenesis, muscles treated with functionalized hydrogels had significantly elevated *Vwf, Vegfa* and Flt1 levels compared to controls (Figure 21B).
- For neurogenesis, muscles in both functionalized hydrogel groups showed higher expression of *Lrp4,* and *Dok7* than controls. Additionally, R4-IL4-treated muscles had higher *Lrp4* and *Rapsyn* than the R1-SDF1 group (Figure 21C), hinting that IL-4 may particularly benefit neuromuscular junction formation.

To sum up, the transplantation of either IL-4 or SDF-1 functionalized hydrogel into acutely injured muscle was beneficial. Both treatments supported enhanced myogenic differentiation (leading to efficient muscle reconstruction), and also promoted angiogenesis and reinnervation of the regenerating muscle. These molecular improvements correlate with the functional improvements observed (especially for R1-SDF1 hydrogel in treadmill tests).

### Hydrogels with Myoblast Co-Transplantation (Acutely Injured Muscle)

We then asked whether adding human iPSCs-derived myoblasts to the hydrogels could further improve outcomes in the injured muscle. For these experiments, injured SCID mouse muscles received R, R1-SDF1, or R4-IL4 hydrogels with either DMD or LMNβ1 myoblasts.

After 28 days, a treadmill test was performed. Muscles co-transplanted with R1-SDF + DMD myoblasts showed the best performance, with significantly longer running times/distances compared to injured untreated controls (Figure 22A).

Histologically, muscles treated with hydrogels + cells regenerated normally. There were no significant differences in fiber CSA, fiber diameter, or percentage of centrally nucleated fibers among any of the hydrogel+cell groups or the control (injured untreated) group (Figure 22B-D). Fibrosis levels remained uniformly low across all groups (no adverse fibrosis noted) (Figure 22E). Moreover, the proposed therapeutic approach did not impact mice body weight nor the weight of treated and contralateral muscle (Figure 22F-H). These findings suggest that introducing cells did not disrupt the regenerative process - all muscles healed with similar structure, and co-delivery of cells did not cause any structural abnormalities.

We next examined gene expression markers in these co-transplanted muscles.
- All muscles that received a hydrogel + DMD myoblasts showed a significantly higher expression of *Myh3* (embryonic myosin heavy chain) compared to any other group (including those with LMNβ1 cells) (Figure 23). This indicates an enhancement of the regenerative (embryonic) muscle fiber program specifically when DMD cells were present.
- Muscles with DMD cells + R hydrogel had elevated *Myh7* and *Pax7* compared to other groups, and muscles with DMD cells + R4-IL4 hydrogel also showed elevated *Myh7.* Notably, any muscle that received hydrogel + cells (of either type) had a significantly higher *Dystrophin* level than intact or untreated muscles (Figure 23). This implies that the human DMD myoblasts (which express human dystrophin) were contributing to dystrophin levels and/or the hydrogels improved mouse dystrophin retention in regenerating fibers.
- For angiogenesis, elevated levels of Vegfa and Flt1 were particularly observed in the muscles treated with R4-IL4 + DMD cells compared to all other tested variants. Moreover, R4-IL4 but with LMNβ1 cells significantly increased Vwf expression (Figure 24A).
- For neurogenesis markers, there were clear improvements: muscles treated with DMD cells + R4-IL4 hydrogel had significantly higher expression of *Rapsyn, Musk,* and Achr compared to others (Figure 24B). Also, *Achr and Lrp4* were elevated in muscles with DMD cells + R1-SDF1 hydrogel.

These results indicate that co-transplantation of human myoblasts (particularly the DMD line) can further enhance certain aspects of regeneration in injured muscles. In our experiments, adding DMD cells to the hydrogels boosted myogenesis (e.g., more Myh3, Myh7, Pax7, Dystrophin), angiogenesis (higher Vegfa, Flt1) and neurogenesis (higher Achr, Musk, Rapsyn). In other words, the cells provided an added benefit beyond the hydrogel alone in the acute injury setting.

### Hydrogels with/without Myoblasts in Dystrophic Muscle (SCID/mdx Mice)

We next evaluated the treatments in the dystrophic muscle model (SCID/mdx mice). Hydrogels (R, R1-SDF1, R4-IL4) were injected into the TA muscles of SCID/mdx mice either alone or with DMD or LMNβ1 myoblasts. The baseline in this model is a chronically regenerating dystrophic muscle.

After 28 days, treadmill tests in the dystrophic mice showed no significant differences among any of the groups (whether treated or not) (Figure 25A). This contrasts with the injury model, likely because dystrophic mice have more variable or subtle functional deficits that were not markedly rescued by these single treatments in the short term.

Histologically, all treated dystrophic muscles appeared normal in overall structure (no overt pathology beyond what is typical for dystrophic muscle).

Detailed analysis of muscle structure and CSA revealed numerous significant differences between muscle groups, especially in the case of the smallest (CSA from 0-749 µm) and the largest fibers (CSA above 3750 µm). Dystrophic muscles injected with hydrogels had more smallest fibers and less large than the intact muscles (Figure 25B). A similar effect was observed in the case of muscles injected with hydrogels and LMNβ1 cells (Figure 25D). In the case of muscles injected with hydrogels and DMD cells, more smallest fibers were observed for muscles that received R and DMD than for all other variants (i.e., intact, R1-SDF1 and DMD, R4-IL4 and DMD) (Figure 25C). The percentage of regenerating fibers with central nuclei was high in all dystrophic groups (as expected due to ongoing cycles of degeneration/regeneration). There were some variations: muscles injected with R or R4-IL4 hydrogels alone, those with DMD cells + R1-SDF1 or R4-IL4, and those with LMNβ1 cells + R4-IL4 all showed elevated percentages of centrally nucleated fibers compared to some other groups (Figure 25E). However, since all dystrophic muscles inherently have many regenerating fibers, these differences are harder to interpret. Importantly, none of the treated dystrophic muscles exhibited increased fibrosis; fibrosis areas remained comparable across all groups (and generally higher than in healthy muscle, but typical for dystrophic muscle) (Figure 25F).

Gene expression analysis in the dystrophic muscles revealed some notable findings:
- The R1-SDF1 hydrogel (without cells) emerged as particularly effective in the dystrophic setting. Muscles treated with R1-SDF1 alone had the highest expression of several regeneration markers: significantly elevated *Myog, Myh2, Myh3* and *Pax7* (myogenesis markers), as well as increased *Cd31, Vwf* (angiogenesis), and *Achr* (neurogenic marker), when compared to other groups (Figure 26A-C).
- In general, adding cells (DMD or LMNβ1) to the hydrogels did not further raise these markers in dystrophic muscles; in fact, the largest improvements were seen with the hydrogel alone (especially R1-SDF1). For instance, none of the cell-containing groups surpassed R1-SDF1-alone in those key markers listed above.
- The R4-IL4 hydrogel, whether alone or with cells, did show one clear benefit: an increased number of regenerating fibers (centrally nucleated fibers) in the treated dystrophic muscles (as noted above, Figure 25E). However, at the molecular level, R4-IL4 (alone or with cells) did not match R1-SDF1's enhancement of gene expression markers.

In summary, in the dystrophic muscle model, the addition of cells to the hydrogels did not confer an obvious advantage beyond the hydrogel treatment alone. This is in contrast to the acute injury model, where adding cells provided extra benefits. The most profound effect in dystrophic muscle was achieved by the R1-SDF1 hydrogel on its own, which significantly boosted myogenic, angiogenic, and neurogenic marker expression. The R4-IL4 hydrogel (with or without cells) did improve one aspect (the quantity of regenerating fibers), but did not significantly elevate the molecular regeneration markers beyond control levels. These results suggest that the dystrophic environment may be less responsive to co-transplanted cells in the short term, or that the functionalized hydrogels already maximized the regenerative stimulus such that added cells provided no further immediate boost. It is possible that a longer observation period might reveal additional benefits of the co-transplantation in dystrophic muscle, and we plan to investigate longer-term outcomes in future studies.

### Overall Findings

In summary, transplantation of the SDF-1-functionalized hydrogel (R1-SDF1) significantly enhanced the functional recovery of injured skeletal muscle, as evidenced by improved treadmill performance. Both types of peptide-functionalized hydrogels (R1-SDF1 and R4-IL4) supported muscle regeneration in vivo by promoting myogenesis (e.g., increased expression of Myh7, Myog, Dmd), angiogenesis (increased Vwf, Vegfa, Flt1), and neurogenesis (increased Lrp4, Dok7) in injured muscles. Co-transplantation of human iPSCs-derived myoblasts provided an additional boost in the injured muscle model: functionalized hydrogels with cells led to further enhancements in myogenesis and neurogenesis (for example, R1-SDF1 + cells increased Myh3, Dmd, Achr and Lrp4; R4-IL4 + cells increased Myh3, Myh7, Dmd, Pax7, Vwf, Vegfa, Flt1, Achr, Musk, Lrp4, Dok7 and Rapsyn). The presence of cells also benefited muscles treated with the unmodified R hydrogel, improving expression of myogenic markers (Myh3, Myh7, Pax7, DMD) and neurogenic marker (Lrp4). In dystrophic muscles, the R1-SDF1 hydrogel alone elicited the greatest response, elevating myogenesis-, angiogenesis-, and neurogenesis-related genes (Myh2, Myh3, Myog, Pax7, Cd31, Vwf, Lrp4 and Achr). Adding myoblasts to the hydrogels in dystrophic muscle did not further increase these benefits within the 28-day period, highlighting that the hydrogel alone was sufficient to induce substantial regeneration in that context. Overall, these peptide-functionalized hydrogels show promise for enhancing muscle regeneration by improving myoblast migration and fusion, supporting the viability and function of transplanted myogenic cells, and stimulating angiogenic and neurogenic processes in regenerating muscle tissue.

### References

[1] Mierzejewski B et al. Human and mouse skeletal muscle stem and progenitor cells in health and disease. Semin Cell Dev Biol. 2020;104:93-104.
[2] Langridge B et al. Regenerative medicine for skeletal muscle loss: a review of current tissue engineering approaches. J Mater Sci Mater Med. 2021;32:15.
[3] Munoz-Canoves P et al. Understanding muscle regenerative decline with aging: new approaches to bring back youthfulness to aged stem cells. FEBS J. 2020;287:406-16.
[4] Eugenis I et al. Cells, scaffolds, and bioactive factors: Engineering strategies for improving regeneration following volumetric muscle loss. Biomaterials. 2021;278:121173.
[5] Ahmad SS et al. Therapeutic applications of biological macromolecules and scaffolds for skeletal muscle regeneration: A review. Int J Biol Macromol. 2024;267:131411.
[6] Namjoo AR et al. Tissue engineering modalities in skeletal muscles: focus on angiogenesis and immunomodulation properties. Stem cell research & therapy. 2023;14:90.
[7] Borselli C et al. Functional muscle regeneration with combined delivery of angiogenesis and myogenesis factors. Proc Natl Acad Sci U S A. 2010;107:3287-92.
[8] Horsley V et al. IL-4 acts as a myoblast recruitment factor during mammalian muscle growth. Cell. 2003;113:483-94.
[9] Lafreniere JF et al. Interleukin-4 improves the migration of human myogenic precursor cells in vitro and in vivo. Exp Cell Res. 2006;312:1127-41.
[10] Chang YH et al. Interleukin-4 Promotes Myogenesis and Boosts Myocyte Insulin Efficacy. Mediators Inflamm. 2019;2019:4182015.
[11] Brzoska E et al. Sdf-1 (CXCL12) induces CD9 expression in stem cells engaged in muscle regeneration. Stem cell research & therapy. 2015;6:46.
[12] Julier Z et al. Promoting tissue regeneration by modulating the immune system. Acta Biomater. 2017;53:13-28.
[13] Kapp TG et al. A Comprehensive Evaluation of the Activity and Selectivity Profile of Ligands for RGD-binding Integrins. Scientific reports. 2017;7:39805.
[14] Michalska et al. In Vitro Bioactivity Evaluation of IL-4 and SDF-1 Mimicking Peptides Engineered to Enhance Skeletal Muscle Reconstruction. J Biomed Mater Res A. 2025;113:e37898.
[15] Pulik L, Mierzejewski B, Ciemerych MA, Brzoska E, Legosz P. The Survey of Cells Responsible for Heterotopic Ossification Development in Skeletal Muscles-Human and Mouse Models. Cells. 2020;9.

## Claims

1. A peptide-based hydrogel for skeletal muscle regeneration, comprising:
a) a self-assembling RADA16-I peptide forming a nanofibrous hydrogel matrix; and
b) at least one bioactive peptide selected from:
peptide fragment of stromal cell-derived factor 1 (SDF-1): SEQ ID NO: 1, SEQ ID NO: 2, SEQ ID NO: 5, SEQ ID NO: 6, or
peptide fragment of interleukin-4 (IL-4): SEQ ID NO: 3, SEQ ID NO: 4, SEQ ID NO: 7, SEQ ID NO: 8,
wherein the bioactive peptide is covalently linked to the RADA16-I sequence via a matrix metalloproteinase (MMPs) cleavable peptide linker and wherein cleavage of the linker by an MMPs results in controlled, localized release of the bioactive peptide in the microenvironment of regenerating muscle tissue.

2. The hydrogel according to claim 1, wherein the bioactive peptide is SEQ ID NO: 1.

3. The hydrogel according to claim 1, wherein the bioactive peptide is SEQ ID NO: 8.

4. The hydrogel according to any claim from 1 to 3, wherein the matrix metalloproteinase cleavable peptide linker is selected from:
- the MMP-7-sensitive linker VLGL, or
- the MMP-2 or MMP-3 or MMP-9 or MMP-13 or MMP-14 sensitive linker GGG-PAGL.

5. The hydrogel according to any claim from 1 to 4, wherein the linker is VLGL and the hydrogel is an R1-type hydrogel.

6. The hydrogel according to any claim from 1 to 4, wherein the linker is GGG-PAGL and the hydrogel is an R4-type hydrogel.

7. A composition comprising: (a) the hydrogel according to any claim from 1 to 6, and (b) myoblasts derived from human pluripotent stem cells, for implantation into damaged skeletal muscle tissue.

8. The peptide-based hydrogel according to any claim from 1 to 6 or the composition according to claim 7 for use as a medicament.

9. The peptide-based hydrogel according to any claim from 1 to 6 or the composition according to claim 7 for use in the treatment of skeletal muscle injury or degeneration.

10. The peptide-based hydrogel according to any claim from 1-6 or the composition according to claim 7 for use in the treatment of a condition selected from: acute skeletal muscle injury, volumetric muscle loss (VML), muscular dystrophy, age-related sarcopenia, cachexia-associated muscle wasting, post-surgical or post-traumatic muscle regeneration, muscle atrophy due to
